Europäisches Patentamt

European Patent Office

Office européen des brevets

(11) Publication number: **0 583 675 A1**

# EUROPEAN PATENT APPLICATION

(21) Application number: **93112393.9**

(22) Date of filing: **19.11.86**

(51) Int. Cl.⁵: **C12N 15/74**, C12N 15/31, A01N 63/00

Diese Anmeldung ist am 02 - 08 - 1993 als Teilanmeldung zu der unter INID-Kode 60 erwähnten Anmeldung eingereicht worden.

(30) Priority: **20.11.85 US 799999**

(43) Date of publication of application: **23.02.94 Bulletin 94/08**

(60) Publication number of the earlier application in accordance with Art.76 EPC: **0 245 489**

(84) Designated Contracting States: **AT BE CH DE FR GB IT LI LU NL SE**

(71) Applicant: **CROP GENETICS INTERNATIONAL, N.V.**
**7170 Standard Drive**
**Dorsey Maryland 21076(US)**

(72) Inventor: **Carlson, Peter S.**
**1909 Westfield Street**
**Alexandria, Virginia 22308(US)**

(74) Representative: **Patentanwälte Grünecker, Kinkeldey, Stockmair & Partner**
**Maximilianstrasse 58**
**D-80538 München (DE)**

(54) **A method of producing hybrid-agricultural-chemical-producing microorganisms.**

(57) A method for producing a hybrid agricultural-chemical producing microorganism capable of entering into endosymbiotic relationship with plants is disclosed.

The microorganisms are prepared by combining genetic material from an agricultural-chemical-producing microorganism and a plant infecting microorganism to form hybrid microorganisms and selecting from the hybrid microorganisms those hybrid microorganisms which are capable of producing an agricultural chemical, which do not create manifestations of disease in the plant host, and which are capable of entering into an endosymbiotic relationship with the plant host. Methods of use are also disclosed.

Fig. 1

Rank Xerox (UK) Business Services
(3.10/3.09/3.3.4)

This application is a continuation-in-part of application Serial No. 534,071, filed September 20, 1983 which is a continuation-in-part of application serial no. 484,560 filed April 13, 1983.

The present invention relates to agricultural-chemical-producing microorganisms, particularly agricultural-chemical-producing bacteria capable of entering into endosymbiotic relationships with host plants whereby the bacteria provide some or all of the plant's agricultural-chemical requirements.

BACKGROUND OF THE INVENTION

Modern commercial agriculture is heavily dependent on the use of agricultural chemicals to enhance the performance of plants. Most notably, chemical fertilizers, particularly fixed-nitrogen fertilizers, are the most common limiting nutrient in crop productivity and often are the most expensive single input for the farmer. Other agricultural chemicals are widely used at great expense to kill plant pests, to prevent disease, or to improve the growing environment of the plant. Still other agricultural chemicals may be added to growing plants or harvested crops to diminish or enhance natural properties or to alter or improve the appearance or sensory appeal of the plant or crop. Agricultural chemicals include, as well, natural or synthetic plant growth regulators, including hormones and the like. Such chemicals are well known to those having ordinary skill in the agricultural art and are hereinafter generically referred to as "agricultural chemicals."

Microorganisms, such as bacteria, fungi, and algae are a natural source of agricultural chemicals. For example, leguminous plants, such as soybeans, alfalfa and clover, derive some of their fixed nitrogen requirements through symbiotic relationships with bacteria of the genus Rhizobium. Particular species of Rhizobium infect the roots of leguminous plants forming nodules in which the bacteria are shielded from oxygen and provided with carbohydrate nutrients. In this anaerobic process, the rhizobia fix atmospheric nitrogen, which is then available for use by the plant.

Microorganisms have also become sources of agricultural chemicals synthesized or prepared by man and applied by spraying or the like onto fields, growing plants, or harvested crops. Antifungal antibiotics, antibacterial antibiotics, and insecticidal antibiotics, for example, have been produced by various bacteria. Antifungal antibiotics include blasticidin S which is produced by Streptomyces griseochromogenes, kasugamycin which is produced by Streptomyces kasugaenis, and polyoxins which are produced by Streptomyces cacaoi var. asoensis. Antibacterial antibiotics include streptomycin produced by Streptomyces qriseus, and tetracycline produced by Streptomyces viridifaciens. Insecticidal antibiotics include tetranactin, produced by Streptomyces aureus strain S-3466, and the beta-exotoxin and delta-endotoxins produced by Bacillus thurinqiensis. See K. Aizawa, "Microbial Control of Insect Pests," and T. Misato and K. Yoneyama, "Agricultural Antibiotics" in Advances in Agricultural Microbiology, N.S.S. Rao, Editor (1982), which is specifically incorporated herein by reference in its entirety.

Similarly, microorganisms have become a source of antibiotics for herbicide use. For example, cycloheximide, produced by Streptomyces qriseus, and herbicidin A and B, produced by Streptomyces saganoensis, exhibit herbicidal activity. See Y. Sekizawa and T. Takematsu, "How to Discover New Antibiotics for Herbicidal Use" in Pesticide Chemistry, J. Miyamoto and P.C. Kearney, Editors (1983), which is specifically incorporated herein by reference in its entirely.

Moreover, microorganisms are known to produce plant growth regulating substances such as various vitamins, auxins, cytokinins, gibberellin-like substances and other stimulating or inhibiting substances. Brown, in her work on "Seed and Root Bacterization" cited below, attributes the production of such substances to species of Azotobacter, Pseudomonas and Bacillus, including B. meqaterium, and B. subtilis.

Bacteria have been found that produce agents active against a number of invertebrates, such as plant pathogenic nematodes. One of these agents is Streptomyces avermitilis, which produces avermectins. S. avermitilis and the antiparasitic compounds it produces are described in U.S. patents 4,310,519, 4,429,042, and 4,469,682, which are specifically incorporated herein by reference in their entirety.

Microorganisms have also been found to produce antiviral antibiotics, including laurusin, which has been isolated from Streptomyces lavendulae, and miharamycin, which has been isolated from Streptomyces miharuensis. See T. Misato, K. Ko, and I. Yamaguchi, "Use of Antibiotics and In Ariculture" in Advances in Applied Microbiology, Vol. 21, (1977), which is specifically incorporated hereby by reference in its entirety.

Other types of bacteria are known to have the ability to solubilize phosphate that is otherwise unavailable to plants. These includes those bacteria belonging to the genera Bacillus and Pseudomonas. Attempts to utilize this ability of these bacteria by inoculating seeds or plants with the bacteria have produced inconsistent results. See, N.S.S. Rao, "Phosphate Solubilization by Soil Microorganisms" in Advances in Aricultural Microbiology, N.S.S. Rao, Editor (1982), which is specifically incorporated herein by reference in its entirety.

Few symbiotic associations between agricultural-chemical-producing microorganisms and major crop species, such as wheat and corn, have been described in the scientific literature. The development of symbiotic relationships between such microbial species and various crops would have many advantages over man-made synthesis and application of the chemical. The advantages of such associations are particularly apparent in the areas of insecticides and fixed-nitrogen fertilizers, which are hereinafter discussed in detail as exemplary of the advantages attainable through the practice of the present invention. It should be understood however, that the teachings contained herein regarding bacterial production of insecticides and of fixed-nitrogen fertilizers may be extrapolated by those having ordinary skill in the art to other bacterially or microbially produced agricultural chemicals, such as those discused above.

A particular advantage of the present invention is that the agricultural chemical is introduced within the entire plant or parts thereof rather than just on the surface as in the case when certain agricultural chemicals are sprayed on the plant. For example, insecticides that are sprayed on and do not penetrate into the plant can be ineffective or of only limited effectiveness against insects that bore into the plant. However, the insecticide should be effective when applied as a hybrid, endosymbiotic microoganism of the present invention.

It is estimated that development of a symbiotic relationship between nitrogen-fixing bacteria and major crop species capable of providing even a minor fraction, e.g., 10-20%, of the fixed nitrogen requirements of non-leguminous crop plants, would produce an economically important substitution of photo-synthetically-derived energy for fossil fuel energy now expended for production of nitrogen fertilizer. Currently, the major sources of fixed nitrogen for non-leguminous economic crop plants are man-made products such as anhydrous ammonia, inorganic nitrogen salts and synthetic organic compounds. Present annual consumption of nitrogen fertilizer in the United States is estimated to be 12 million tons. Such nitrogen fertilizers are largely prepared by energy intensive processes for the conversion of atmospheric nitrogen into ammonia.

A major thrust of nitrogen-fixation research in recent years has been directed towards the isolation, characterization, transformation and expression in plants of the nitrogen-fixing genes from rhizobia and other nitrogen-fixing bacteria by genetic modification of the plants themselves. This technique requires a complete understanding of the genetics and biochemistry of nitrogen fixation which, it is estimated, is 10 to 15 years or more in the future. See generally, J.E. Beringer, "Microbial Genetics and Biological Nitrogen Fixation," in Advances in Agricultural Microbiology, N.S.S. Rao, Editor (1982), and G.P. Roberts et al., "Genetics and Regulation of Nitrogen Fixation," Ann. Rev. Microbiol., 35:207-35 (1981), both of which are specifically incorporated herein by reference in their entirety.

Other types of bacteria besides Rhizobium are known to have the capability of fixing atmospheric nitrogen. These include those belonging to the genera Azotobacter, Azomonas, Derxia and Beijerinckia, which fix nitrogen aerobically, and those belonging to the genus Klebsiella which, like Rhizobium, fix nitrogen anaerobically. The literature is filled with reports of unsuccessful attempts to develop symbiotic relationships, similar to Rhizobium-legume relationships, between these bacteria and non-leguminous plants.

In the early 1940's, for example, it was proposed in the Soviet Union and elsewhere that nitrogen-fixing bacteria could be used as soil inoculants to provide some or all of the fixed nitrogen requirements of non-leguminous crop plants. Indeed, claims of increased crop yield associated with such inoculations were widespread. Critical analysis of the early yield results, however, related that positive significant effects occurred in only about one-third of the trials. It was later established that yield increases associated with bacterial inoculation, including inoculation with species of Azotobacter, are primarily due to changes in the rhizosphere microbial population, disease suppression by the inoculants, production of plant growth promoting substances, and mineralization of soil phosphates. No ability to establish an associative symbiotic relationship with nitrogen-fixing bacteria in non-leguminous plants had been demonstrated. See M.E. Brown, "seed and Root Bacterization," Annual Review of Phytopathology, 12:181-197 (1974), which is specifically incorporated herein by reference in its entirety.

It has been demonstrated that an artificial symbiosis can be created between non-leguminous plant cells in tissue culture and aerobic nitrogen-fixing bacteria such as Azotobacter vinelandii. The demonstration involved forced association between higher plant and bacterial cells and did not provide a technique whereby a symbiotic association between such nitrogen-fixing bacteria and growing plants could be created. See P.S. Carlson, et al., "Forced Association Between Higher Plant and Bacterial Cells In Vitro," Nature, Volume 252, No. 5482, pp. 393-395 (1974), which is specifically incorporated herein by reference in its entirety.

Another effort to create higher organisms capable of fixing their own nitrogen, for example, involved attempts to create new organelles in higher organisms by similar forced association of lower forms of organisms having nitrogen-fixing abilities. Such efforts to parallel the theoretical biological development of known organelles have included, for example, the efforts of Burgoon and Bottino to induce the uptake of nitrogen-fixing Gloecapsa sp. blue-green algae by plant cells, the efforts of Davey and Cocking to induce the uptake nitrogen-fixing Rhizobium sp. bacteria into plant cells, and the efforts of Giles to induce uptake of nitrogen fixing Azotobacter vinelandii bacteria by ectomycorrhizal fungi. See A.C. Burgoon and P.J. Bottino, Journal of Heredity, Volume 67, pp. 223-226 (1972); M.R. Davey and E.C. Cocking, Nature, Volume 239, pp. 455-456 (1972); and K.L. Giles, "The Transfer of Nitrogen-Fixing Ability to Nonleguminous Plants," Plant Cell and Tissue Culture Principles and Applications, W.R. Sharp et al., Editors (1979). Neither the work of Burgoon and Bottino nor the work of Davey and Cocking was successful in generating a nitrogen-fixing higher organism. If the effects of Giles were successful, which has not been conclusively established, they would at best result in fungi with organelle-like structures capable of fixing nitrogen which are capable only of living on the outsides of roots of forest species rather than living endosymbiotically with major crop plants.

Another attempt to create a symbiotic relationship between nitrogen-fixing bacteria and non-leguminous plants involved the screening and selection of plant hybrids in an effort to identify some plant varieties which were capable of associating with aerobic fixing bacteria, such as Azotobacter vinelandii, in the soil. However, those efforts resulted in the identification of plant species which were only about .5% as active as soybean plants inoculated with rhizobia which were not deemed to be commercially useful. See S.W. Ela, et al., "Screening and Selection of Maize to Enhance Associative Bacterial Nitrogen Fixation," Plant Physiol., 70:1564-1567 (1982), which is specifically incorporated herein by reference in its entirety.

The numerous unsuccessful attempts to create associative symbiotic relationships between nitrogen-fixing bacteria and non-leguminous plants have resulted in the development of numerous techniques for genetic manipulation and analysis. These attempts have also resulted in rather extensive characterization of the genetic makeup of nitrogen-fixing bacteria such as Azotobacter. For example, it has been discovered that mutant strains of Azotobacter vinelandii lacking the nitrogen-fixation gene could be transformed into bacteria having the capability to fix nitrogen by introduction of genetic material from Rhizobium species. See G.P. Roberts, "Genetics and Regulation of Nitrogen Fixation," Ann. Rev. Microbiol., 35:207-35 (1981). Moreover, new techniques for protoplast fusion, which involve promotion of interspecific genetic recombination by fusing two different cells which have had their cell walls removed, were recognized as providing a potentially attractive technique for the production of new strains for commercial purposes without requiring an understanding of the underlying genetics. It was acknowledged in the scientific literature, however, that it has yet to be determined whether or not protoplast fusion can be used for genetic studies of gram-negative nitrogen-fixing bacteria. See J.E. Beringer, "Microbial Genetics and Biological Nitrogen Fixation," Advances in Agricultural Microbiology, N.S.S. Rao, Editor (1982) at 13.

Indeed, experimentation related to protoplast fusion of nitrogen-fixing bacteria has, prior to the present invention, failed to produce evidence that non-pathogenic endosymbiotic relationships between higher plants and fusion products of nitrogen-fixing bacteria can be produced. For example, Du Qian-you and Fan Cheng-ying, "A Preliminary Report on the Establishment of Nitrogen Fixation System in the Crown Gall of Tomato Plant," Acta Bontanica Sinica, Vol. 23, No. 6 (1981), which is specifically incorporated herein by reference in its entirety, described an attempt to effect protoplast fusion between parent strains of Aqrobacterium tumefaciens (Pen$^r$, Ti) and Azotobacter chroococcum (Pen$^s$, Nif$^+$). The hybrids were selected for nitrogen fixation and their resistance to penicillin and were thereafter inoculated on the stem of a tomato plant. The inoculation produced crown galls or tumors found to possess nitrogenase activity under aerobic conditions according to the acetylene reduction test. The fusion products of the experiment produced a pathogenic response in the host plant and were not shown to be capable of entering into an endosymbiotic relationship with the plant. Specifically, it could not be ascertained whether the fixed nitrogen being produced in the vicinity of the tumors was being used by the plant as a source of nitrogen. Finally, it could not be determined whether the nitrogenase activity observed was the result of a pathogenic hybrid or of a penicillin resistant mutant of Azotobacter remaining in a mixed inoculum. Moreover, it has now been discovered that the attempts of Du Qian-you et al. to mimic the nodule formation mechanism of the Rhizobium-legume interaction by intentional inclusion of the tumor-forming Ti plasmid in their hybrids were headed in the opposite direction from that required to produce hybrid bacteria capable of entering into successful endosymbiotic relationships with plant hosts.

Other unsuccessful attempts to extend the range of nitrogen-fixing Rhizobium species include the insertion of the Ti plasmid from Aqrobacterium tumefaciens into selected Rhizobium strains. See P.J.J. Hooykass, et al., J. Gen. Microbi., 98:477-484 (1977), which is specifically incorporated herein by reference in its entirety. These studies resulted in Rhizobium strains able to produce galls on non-leguminous dicotyledenous plants which will fix nitrogen but only under anaerobic conditions not naturally occurring in such plants. The criticisms outlined in the previous paragraph also applies to this work.

Despite substantial economic incentive to do so, the prior art has been unable to prepare hybrid nitrogen-fixing bacteria capable of entering into endosymbiotic relationships with non-leguminous plant hosts or any other hybrid agricultural-chemical-producing microorganisms capable of entering into endosymbiotic relationships with plant hosts.

SUMMARY OF THE INVENTION

A method of producing hybrid agricultural-chemical-producing microorganisms capable of entering into endosymbiotic relationships with a plant host has been discovered which comprises combining genetic material of an agricultural-chemical-producing microorganism and a plant-infecting microorganism which infects the plant host (hereinafter sometimes referred to as an "infecting microorganism") to form hybrid microorganisms and selecting from the hybrid microorganisms those hybrid microorganisms which are capable of producing an agricultural chemical and of entering into an endosymbiotic relationship with the plant host and which do not create manifestations of disease in the plant host.

The hybrid microorganisms are formed by combining some or all of the genetic material of the agricultural-chemical-producing microorganism and the infecting microorganism. The genetic material is combined through use of various techniques, such as recombinant DNA, recombinant RNA, cell fusion, conjugation, plasmid transfer, transformation, transfection, and microinjection.

The microorganisms resulting from the process of the present invention may be introduced into crop plants by a variety of means, including injection and may be employed to coat agricultural seed, to infect agricultural seed, in the preparation of a soil drench, and the like. The hybrid microorganisms of the present invention, upon association with crop plants, are capable of supplying some or all of the plants' agricultural chemical needs. Agricultural chemicals that can be produced by hybrid bacteria in accordance with the present invention include fertilizers, particularID fixed-nitrogen fertilizers; antibiotics, including antibacterial, antiviral, antifungal, insecticidal, nematocidal, miticidal, and herbicidal agents; plant growth regulators, including plant hormones, and the like.

In a preferred embodiment, there has been discovered a method of producing hybrid agricultural-chemical-producing microorganisms capable of entering into endosymbiotic relationships with a plant host comprising:

(A) in any convenient order;
   (1) identifying a plant host;
   (2) identifying an infecting microorganism which infects the plant host;
   (3) selecting mutants of the infecting microorganism with one or more selectable traits in addition to ability to infect the host; and
   (4) selecting an agricultural-chemical-producing microorganism having one or more selectable traits;
(B) forming fusion hybrids of the infecting microorganism and the agricultural-chemical-producing microorganism;
(C) selecting serially from the products of the previously performed step or substep and in any convenient order:
   (1) a subgroup comprising those hybrids having both at least one of the selectable traits of the agricultural-chemical-producing microorganism and at least one of the selectable traits of the infecting microorganism;
   (2) a subgroup comprising those hybrids which manifest the ability to interact with plant tissue in the manner in which the infecting microorganism interacts with plant tissue during the initial phase of infection in the plant host;
   (3) a subgroup comprising those hybrids which, upon application to the host, do not create manifestations of a disease; and
   (4) a subgroup comprising those hybrids having the ability to produce the agricultural chemical if not previously selected for; and
(D) selecting hybrid agricultural-chemical-producing microorganisms capable of entering into endosymbiotic relationships with a plant host by selecting from the products of the last performed step of stages (C)(1) to (C)(4) those hybrid microorganisms capable of improving the performance of the plant host under conditions wherein its performance could be improved by direct application of the agricultural chemical or agricultural-chemical-producing microorganism to the plant.

In a particularly preferred embodiment, the hybrid microorganisms are hybrid bacteria produced by protoplast or spheroplast fusion of an infecting bacterium having the same response to gram stain. Certain gram-positive bacteria, notably members of the genus Bacillus, Streptomyces, and Clavibacter are particularly useful for the formation of the preferred hybrid bateria.

In another preferred embodiment, the hybrid agricultural-chemical-producing microorganism of the present invention capable of entering into endosymbiotic relationships with a plant are formed by the steps comprising:

(A) in any convenient order:

(1) identifying the plant host; and

(2) identifying an infecting microorganism which infects the plant host;

(B) preparing a vector capable of being transferred into and replicating in the infecting microoganism;

(C) Preparing an expression module capable of directing the production of an agricultural chemical by the infecting microorganism;

(D) placing the expression module in the vector to create an expression vector capable of being transferred into and replicating in the infecting microorganism, the expression vector being capable of directing the production of the agricultural chemical by the infecting microorganism;

(E) Transforming the infecting microorganism with the expression vector to produce hybrid microorganism;

(F) selecting for the hybrid microorganism;

(G) selecting serially from the selected hybrid microorganisms and in any convenient order:

(1) a subgroup comprising those hybrid microorganisms which manifest the ability to interact with plant tissue in the manner in which the infecting micro-organism interacts with plant tissue during the initial phase of infection in the plant host;

(2) a subgroup comprising those hybrid microorganisms which, upon application to the host, do not create manifestations of disease; and

(3) a subgroup comprising those hybrid microorganisms having the ability to produce the agricultural chemical if not previously selected for; and

(H) selecting hybrid agricultural-chemical-producing micro-organisms capable of entering into endosymbiotic relationships with a plant host by selecting from the products of the last performed step of steps (G)(1) to (G)(3) those hybrid microorganisms capable of improving the performance of the plant host under conditions wherein the performance would be improved by direct application of the agricultural chemical or the agricultural-chemical-producing microorganism to the plant host.

In a particularly perferred embodiment, the hybrid agricultural-chemical-producing microorganisms capable of entering into endosymbiotic relationships with a plant host are formed by the steps comprising:

(A) in any convenient order:

(1) identifying a plant host; and

(2) identifying an infecting microorganism which infects the plant host;

(B) preparing an integration vector containing an integration sequence and capable of integration into the genome of the infecting microorganism;

(C) preparing an expression module capable of directing the production of an agricultural chemical by the infecting microorganisms;

(D) placing the expression module within the integration sequence of the integration vector, thereby producing a modified integration vector capable of integrating into the genome of the infecting microorganism and directing the production of the agricultural chemical by the infecting microorganism;

(E) transforming the infecting microorganism with the modified integration vector to produce hybrid microorganisms;

(F) selecting for the hybrid microorganisms;

(G) selecting serially from the selected hybrid microorganisms and in any convenient order:

(1) a subgroup comprising those hybrid microorganisms which manifest the ability to interact with plant tissue in the manner in which the infecting microorganism interacts with plant tissue during the initial phase of infection in the plant host;

(2) a subgroup comprising those hybrid microorganisms which, upon application to the host, do not crease manifestations of disease; and

(3) a subgroup comprising those hybrid microorganisms having the ability to produce the agricultural chemical, if not previously selected for; and

(H) selecting hybrid agricultural-chemical-producing microorganisms capable of entering into endosymbiotic relationships with a plant host by selecting from the products of the last performed step of steps (G,(1) to (G)(3) those hybrid microorganisms capable of improving the performance of the plant host under conditions wherein the performance would be improved by direct application of the agricultural chemical or the agricultural-chemical-producing microorganism to the plant host.

The processes are capable of producing agricultural-chemical-producing microorganisms capable of entering into endosymbiotic relationships with both monocotyledonous and dicotyledonous plants. For dicotyledonous plants, infecting bacteria of the genus Agrobacterium are preferred, with strains of Agrobacterium tumefaciens being particularly preferred. Species of the genus Erwinia, such as Erwinia carotovora may also be used, as may species of the genus Pseudomonas, such as Pseudomonas solanacearum and Pseudomonas syringae, of the genus Xanthomonas, such as Xanthomonas campestris, and of the genus Streptomyces, such as S. impomoea. For monocotyledonous plants, species of the genus Erwinia, such as Erwinia stewartii, are preferred infecting bacteria. Species of the genus Xanthomonas, such as Xanthomonas campestris, species of the genus Azospirillum, such as Azospirillum lipoferum and Azospirillum brasilense, and species of the genus Pseudomonas, such as Pseudomonas syringae are also contemplated as being useful. Pseudomonas syringae is contemplated as being particularly useful as an infecting baterium for the formation of fusion products applicable to cereals, including temperate cereals and rice. Clavibacter species such as C. xyli subsp. xyli and C. xyli subsp. cynodontis are particularly useful for grasses, such as maize, sorghum, and the like.

Preferred agricultural-chemical-producing microorganisms and the chemicals and/or applications for which their metabolic products are useful are identified in Table I below and include organisms having the ability to produce fertilizers, including fixed nitrogen and chemicals capable of solubilizing phosphates, antibiotics, including antibacterial compounds, antifungal compounds, antiviral compounds, insecticides, nematocides, miticides and herbicides, and plant growth regulators. Additionally, useful organisms may be selected or modified to produce other agricultural chemicals, as above defined, including fragrances, antifeeding agents and the like.

The processes of the present invention result in novel, stable hybrid microorganisms having the ability to produce one or more agricultural chemicals and enter into endosymbiotic relationships with a plant host. The nitrogen-fixing hybrid bacteria of the present invention, for example, having been shown to improve the yield of non-leguminous crop plants growing under low fixed nitrogen conditions by amounts of from 10 to 180%, due primarily, it is believed, to fixed nitrogen produced by the hybrids of nitrogen-fixing bacteria nad infecting bacteria in the course of their endosymbiotic relationship with the plant host.

Additional objects and advantages of the invention will be set forth in part in the description which follows, and in part will be obvious from the description, or may be learned from the practice of the invention. The objects and advantages may be realized and attained by means of the instrumentalities and combinations particularly pointed out in the appended claims.

The hybrid agricultural-chemical-producing microorganisms produced by the above-described method may be further modified by natural or artificial genetic techniques to improve their performance as sources of agricultural-chemical for the plant host. Such modification could result, for example, in the ability to excrete the agricultural chemical, such as fixed nitrogen, including the ability to excrete the agricultural chemical even in the presence of adequate amounts of that chemical from other sources; in a reduction of the hybrid's resistance to cold temperature (i.e., to prevent unintended proliferation of the hybrids from year to year); in enhancement of the hybrid's ability to withstand drought, disease, or other physiological stress; in the introduction of additional agricultural-chemical-producing functions; or in modification of the hybrid so that it cannot grow outside the plant host.

While the steps of the above-described method may be carried out in any convenient order, it is desirable that the process of selection for agricultural-chemical-producing ability and for ability to interact with plant tissue in the manner in which the infecting microorganism interacts with plant tissue during the initial phases of infection in the host plant be carried out two or more times before the step relating to selection of those hybrids which do not manifest symptoms of disease in the plant host.

The selectable traits associated with the infecting bacterium may be antibiotic resistant, need for specific nutritional supplementation (auxotrophism), resistance to toxins, or the like. The selectable traits associated with the agricultural-chemical-producing microorganism may be the ability to produce an agricultural chemical alone or in combination with one or more of those traits previously mentioned with respect to the infecting microorganism. The interaction with plant tissue screened for in the above-described method may be, for example, the ability to bind to plant tissue, the ability to spread throughout the vascular system of the plant, or the like. Where the interaction associated with the initial phase of infection is binding to plant cells, it is desirable to limit further the microbial subgroup resulting from the penultimate step by selection of microbial subgroup for further screening in accordance with the above-described process comprising those hybrids which spread most readily throughout the plant host.

By appropriate selection of the infecting microorganism, hybrids may be obtained which are capable of entering into endosymbiotic relationships with cereals, such as wheat, triticale, barley, rye, rice and oats; grasses, such as brome grass, blue grass, tall fescue grass, Fine fescue grass, rye grass, and bermuda grass; tropical grasses, such as sugar cane, corn, millet and sorghum; solanaceous plants, such as potatoes, tomatoes, tobacco, eggplant and pepper; brassicaceous plants such as cauliflower, broccoli, cabbage, kale and kohlrabi; other vegetables, such as carrot and parsley; other agriculturally grown plants, such as sugar beets, cotton, fruit trees, berry plants, and grapes and economically important tree species, such as pine, spruce, fir and aspen. The process of the present invention and the resulting microorganisms may also be used to fulfill some or all of the fixed-nitrogen or other agricultural chemical requirements of leguminous plants, such as soybeans, alfalfa, clover, field beans, mung beans, peas and other pulses, as a supplement, for example, to fixed nitrogen provided by species of Rhizobium associated with nodules of their roots.

BRIEF DESCRIPTION OF THE DRAWINGS

The accompanying drawings, which are incorporated in and constitute a part of this specification, illustrate one embodiment of the invention and, together with the description, serve to explain the principles of the invention.

Figure 1 depicts a partial restriction map of the plasmid pCG300.

Figure 2 depicts a partial restriction map of the plasmid pCG306, which is derived from pCG300.

Figure 3 depicts a partial restriction map of the plasmid pCG6.

Figure 4 depicts a truncated B. thuringiensis delta endotoxin gene fused to a kanamycin resistance gene in the vector mBTK65.

Figure 5 depicts the insertion of a truncated B. thuringiensis delta endotoxin gene fused to a kanamycin resistance gene into pCG6 Neo$^s$ and the insertion of an expression module containing these genes into pCG300.

DESCRIPTION OF THE PREFERED EMBODIMENT

Reference will now be made in detail to the presently preferred embodiments of the invention, which together with the following examples, serve to explain the principles of the invention.

As noted above, the present invention relates to a method of producing hybrid agricultural-chemical-producing microorganisms capable of entering into endosymbiotic relationships with a plant host which comprises combining genetic material from an agricultural-chemical-producing microorganism and an infecting microorganism which infects the plant host to form hybrid microorganisms and selecting from the hybrid microorganisms those capable of producing the agricultural chemical, which do not create manifestations of a disease in the plant host, yet which are capable of entering into an endosymbiotic relationship with the plant host. Preferably, the hybrid agricultural-chemical-producing microorganisms are capable of improving the performance of the plant host under conditions wherein the performance would be improved by direct application of the agricultural chemical or the agricultural-chemical-producing microorganism to the plant. Most preferably, the agricultural-chemical-producing microoganism and the infecting microorganism are bacteria. As used throughout this specification, the term "direct application" means application to the whole plant or any part of the plant, including systemic or partial systemic application.

The term "microorganism" as used herein is intended to encompass bacteria, fungi (including yeast), and algae. The term "bacteria" as used herein is intended to encompass bacteria, bacteria-like organisms and their equivalents, including gram positive bacteria, gram negative bacteria, actinomycetes and the like. The only limitations on the taxomonic classification of the microorganisms used in accordance with the present invention is that some or all of their genetic material be capable of being used to produce viable hybrid organisms expressing phenotypical properties of both parents as more fully described herein.

"Infecting microorganisms" as used throughout this specification is intended to connote only microorganisms which enter and live within the plant and normally produce symptoms of disease but also microorganisms which enter and live within the plant symbiotically or commensally. Indeed, some of the plant infecting microorganisms used in accordance with the present invention, while normally creating pathogenic responses in some plant hosts, will not normally produce such responses in all plant hosts.

Performance can be determined and evaluated by those skilled in the art by considering any one or more of a multitude of factors. These include: 1) resistance to environmental stress, such as drought, high salinity, pests, and harmful chemicals; 2) increased yield; 3) faster maturity; 4) less dependency on added nutrients; and 5) enhanced quality of the plant produce of interest.

The hybrid microorganisms are formed by combining some or all of the genetic material of the agricultural-chemical-producing microorganism and the infecting microorganism. The genetic material is combined by the techniques of recombinant DNA, recombinant RNA, cell fusion, conjugation and plasmid transfer, transformation, transfection transduction, and microinjection. Some of these techniques are described in Maniatis, F., E.F. Fritsch, and J. Sambrook, Molecular Cloning, A Laboratory Manual (Cold Spring Harbor Laboratory 1982) and Miller, J.H., Experiments in Molecular Genetics (Cold Spring Harbor Laboratory 1972), both of which are specifically incorporated herein by reference in their entirety. Selection of a technique by which the hybrid microoganisms of the present invention may be formed will generally be within the capabilities of one of ordinary skill in the art based on the above-referenced scientific literature and teachings disclosed herein.

In a preferred embodiment, the agricultural-chemical-producing microorganisms of the present invention capable of entering into endosymbiotic relationships with a plant host may be formed by steps comprising:

(A) in any convenient order:

(1) identifying the plant host;

(2) identifying an infecting microorganism which infects the plant host;

(3) selecting mutants of the infecting microorganism with one or more selectable traits in addition to ability to infect the host; and

(4) selecting an agricultural-chemical-producing microorganism having one or more selectable traits;

(B) Forming fusion hybrids of the infecting microorganism and the agricultural-chemical-producing microorganism;

(C) selecting serially from products of the previously performed step or substep and in any convenient order;

(1) a subgroup comprising those hybrids having both at least one of the selectable traits of the agricultural-chemical-producing microorganism and at least one of the selectable traits of the infecting microorganism;

(2) a subgroup comprising those hybrids which manifest the ability to interact with plant tissue in the manner in which the infecting microorganism interacts with plant tissue during the initial phase of infection in the plant host;

(3) a subgroup comprising those hybrids which, upon application to the host, do not create manifestations of disease; and

(4) a subgroup comprising those hybrids having the ability to produce the agricultural chemical if not previously selected for; and

(D) selecting hybrid agricultural-chemical-producing microorganisms capable of entering into endosymbiotic relationships with a plant host by selecting from the products of the last performed step of steps (C)(1) to (C)(4) those hybrid microorganisms capable of improving the performance of the plant host under conditions wherein the performance could be improved by direct application of the agricultural chemical or the agricultural-chemical-producing microorganism to the plant.

As used in the steps above, the term "selecting" means any intervention or combination of interventions such that the desired microorganism can be recongized either by its ability to survive or by its unique properties.

As indicated, the steps of the method may be performed in any convenient order. Preferably, they are performed in the order recited with the selectable traits of the agricultural-chemical-producing microorganism screened for in step (C)(1) including the ability of the hybrid to produce the agricultural chemical in question, as in the case of fixed-nitrogen producing bacteria. If screening for ability to produce the agricultural chemical in question is not conducted in the first screening step, then it is preferred to conduct such screening (step C(4)) prior to our concurrently with the screening in the plant host contemplated by steps (C)(2) and (C)(3). When performed in the order recited, the antibiotic resistance markers or the like constituting the selectable traits associated with the infecting bacterium need not survive subsequent screening procedures.

The stable hybrid microorganisms resulting from processes of the present invention are distinguished from hitherto known organisms in that they have both the ability to produce agricultural chemicals and the ability to enter into endosymbiotic relationships with a plant host. The endosymbiotic relationship referred to is one in which the organism actually exists within and spreads throughout all or a portion of the plant host, without causing a pathogenic response, deriving some or all of its energy requirements from carbohydrates and other materials produced by the plant host and providing agricultural chemicals which may be used by the plant host to supplement those otherwise available.

The plant hosts with which the microorganisms of the present invention may establish endosymbiotic relationships may include virtually all economically important plants. In addition, any particular hybrid microorganism is not necessarily limited to one particular species of plant host. Its host range will depend upon many factors, including the infecting microorganism from which it is derived. At the same time, the phenotypic traits controlled by the genetic material of the infecting microorganism from which it is derived will place limits on its host range, as will other traits specifically engineered into the hybrid organism.

The process of the present invention has been shown to be capable of producing stable hybrid microroganisms having the ability to produce an agricultural chemical and of entering into endosymbiotic relationships with both monocotyledonous and dicotyledonous plant hosts. A particularly important group of plant hosts for which the products of the present invention may serve as endosymbiotic agricultural-chemical-producing microorganisms are the cereals, including temperate cereals, such as wheat, triticale, barley, rye, rice and oats. Endosymbiotic agricultural-chemical-producing microorganisms formed in accordance with the present invention may also be useful in supplementing the agricultural chemical, including fixed nitrogen, needs of economically important sod and forage grasses, such as brome grass, blue grass, tall fescue grass and bermuda grass. Similarly, the organisms of the present invention have a demonstrated ability to increase the yields of tropical grasses, such as sugar cane, corn, millet and sorghum. Solanaceous plants, such as potatoes, tomatoes, tobacco, eggplant and pepper are suitable plant hosts to which the organisms of the present invention may be applied, as are brassicaceous plants, such as cauliflower, broccoli, cabbage, kale and kohlrabi. Miscellaneous vegetables such as carrots and parsley; other agriculturally grown plants, such as sugar beets, cotton, fruit trees, berry plants and grapes; and economically important tree species, such as pine, spruce, fir and aspen, may also serve as plant hosts for the organisms or the present invention.

Even though plants may have symbiotic relationships with microorganisms which produce agricultural chemicals, such as leguminous plants with bacteria of the genus Rhizobium which fix atmospheric nitrogen anaerobically in root nodules, these plants are obtained in the greatest yield when provided with supplemental agricultural chemical sources through fertilization or otherwise. Accordingly, it is envisioned that such supplemental agricultural chemicals, including supplemental fixed nitrogen, for such plants nay be provided by microorganisms formed in accordance with the present invention capable of producing agricultural chemicals, including fixed nitrogen, and capable of entering into endosymbiotic relationships with these leguminous plants.

The agricultural-chemical-producing microorganism employed in the present invention may be any microorganism that produces an agricultural chemical or chemical effect of interest. Agricultural-chemical-producing microorganism that may be employed in the present invention are those capable of producing antibiotics, antifungal agents, antiviral agents, insecticides, nematocides, miticides, herbicides, plant growth regulating compounds, fertilizing chemicals other than fixed nitrogen, fragrences, sensory enchancing chemicals, antifeeding agents and the like. Selection of such agricultural-chemical-producing microorganism will be within the capabilities of those skilled in the art in light of the teachings and illustrations herein.

In one embodiment, the agricultural-chemical-producing microorganism is a bacterium capable of fixing atmospheric nitrogen aerobically. Such a bacterium may be selected from the genera Azotobacter, Azomonas, Beijerinckia, and Derxia, among others. A preferred group of nitrogen fixing bacteria are those from the genus Azotobacter, such as Azotobacter vinelandii, Azotobacter paspali, Azotobacter beijerinckia and Azotobacter chroococuum. Azotobacter vinelandii is particularly preferred because its genetics and regulation of nitrogen fixation have been extensively studied and because it has a demonstrated ability to accept and express genetic material from other bacterial genera. See G.P. Roberts, et al., "Genetics and Regulation of Nitrogen Fixation," Ann. Rev. Microbiol., 35:207-35 (1981). Although Azotobacter and other significant aerobic nitrogen fixing bacteria are generally gram-negative bacteria, it is to be understood that the techniques of the present invention are applicable to both gram-positive and gram-negative bacteria.

A table of exemplary agricultural-chemical-producing bacteria comtemplated as useful in accordance with the present invention and the agricultural chemical or chemical effects they produce appear in Table I.

Table I. Agricultural-Chemical-Producing Microorganisms

| Group | Genus | Species | Chemical Application |
|---|---|---|---|
| Antibiotics antifungal agents | Streptomyces | cacaoi var. | produces polyoxins which prevent rice sheath blight |
| | | griseus | produces cycloheximide which prevents onion downy mildew |
| | | griseo-chromagenes | produces blasticidin S which prevents rice blast |
| | | hygrosco-picus var. limoneus | produces val-idamycin A which prevents rice sheath blight |
| | | kasugaensis | produces kasugamycin which prevents rice blast |
| | | kitazawaensis | produces ezomycin which prevents stem rot of kidney beans |
| | | Tendae Tu/901 | produces nik-komycin which is active against various fungi |
| | Pseudomonas | fluorescens strain B10 | causes iron to become unavailable to other micro-ganisms such as fungi |
| | Streptover-ticillium | rimofaciens | produces mild-omycin which is active against various powdery mildews |

| Antibac-terial agents | Streptomyces | chibaensis | produces celo-idin which prevents various bacterial diseases |
|---|---|---|---|
| | | griseus | produces streptomycin which prevents various bacterial diseases |
| | | lavendulae No. 6600 Gc-1 | produces laurusin which prevents bacterial leaf blight of rice plants |
| | | spheroides | produces novobiocin which prevents tomatoe canker |
| | | venezuelae | produces chloram-phenicol which prevents bacterial leaf blight of rice plants |
| | | viridi-faciens | produces tetracycline which prevents various bacterial diseases |
| | Agrobacterium | radiobacteria strain 84 | produces agrocin - 84 |
| | Nocardia | sp. | produces myomycin |
| Antiviral Agents | Streptomyces | hygrosco-picus var. aabomyceticus | produces aabomycin A which inhibits its TMV multiplication in tobacco tissue |
| | | lavendulae No. 6600 GC-1 | produces laurusin which inhibits TMV multiplication in tobacco tissue |
| | | miharuensis | produces miharamycin which is effective in controlling TMV, CMV, PVX, and RSV |

| Insecticides | Bacillus | cereus | affects hymenoptera, lepidoptera, coleoptera |
| --- | --- | --- | --- |
| | | euloomarahae | affects coleopteran larvae, especially, Japanese beetle |
| | | fribougensis | affects coleopteran larvae, especially, Japanese beetle |
| | | lentimorbus | affects coleopteran larvae, especially Japanese beetle |
| | | moritai | affects seedcorn maggot |
| | | popilliae | affects coleopteran larvae, especially Japanese beetle |
| | | thuringiensis var. alesti, dendrolimus, entomocidus, kurstaki, sotto, tenebrionis, israelensis, iberliner | affect numerous insect pest larvae |
| | Clostridium | bravidaciens | affects tent caterpillar |
| | | malacusomae | effects tent caterpillar |
| | Streptomyces | strain B-41-146 | produces milbemycin |
| | | avermitilis | produces avermectins |
| Nematocide | Bacillus | penetrans | effective against root-knot nematodes |
| | Streptomyces | avermitilis | produces avermectins |
| Miticide | Streptomyces | aureus S-3466 | produces tetranactin |
| | | avermitilis | produces avermectins |
| Herbicides | Streptomyces | griseus | produces cycloheximide |

13

| | | saganoensis | produces herbicidin A, B |
|---|---|---|---|
| | | sp. | produces bialaphos |
| | | sp. | produces anisymycin |
| | Pseudomonas | syringae pv. atropurpurea | produces coronatine which causes chocholate spot disease on Italian ryegrass |
| Fertilizers Nitrogen-fixing | Azotobacter | beijerinckia, enroococcum, paspali, vinelandii | fix atomspheric nitrogen |
| | Azomonas | | fix atomspheric nitrogen |
| | Beijerinckia | | fix atomspheric nitrogen |
| | Derxia | | fix atomspheric nitrogen |
| Phosphate Solubi-lizing | Bacillus | circulans, flourescens, megaterium, mesentericus, mycoides, polymyxa, pulvitaciens, subtilis | solublize bound phosphate |
| | Pseudomonas | calcis, liquifaciens, putida, rathonia, striata | solubilize bound phosphates |
| Plant Growth Regulators | Azotobacter | sp. | produce various vitamins, auxins, cytopkinins, gibberellin-like substance and other plant hormones |
| | Agrobacterium | sp. | |
| | Pseudomonas | sp. | |
| | Bacillus | megaterium, subtilis | |

The infecting microorganism capable of infecting the plant host employed in the present invention may be any of a wide variety of bacterial species which infect the plant host under consideration. Preferably, the infecting microorganism should have a known method of interaction with the plant host during the initial phase of infection. The infecting microorganism may be either a pathogen, including latent pathogens, or an endosymbiotic species. In the case of pathogens, it is preferred that the pathogen create a visible

14

manifestation of the disease associated with it. Exemplary pathogens include species of the genera Agrobacterium and Erwinia. Exemplary endosymbiotic species include species of Azospirillum, Corynebacterium, and Clavibacter. The species of Azospirillum are known to live in the roots of tropical grasses, such as sugar cane, and temperate grasses, such as wheat, without causing manifestations of disease. Certain species of Corynebacterium live in wheat and corn. It has been discovered that a species of Clavibacter lives in corn.

Most preferably, the infecting microorganism is a strain which is specific, or nearly specific, to the particular crop plant host under consideration in order to limit the potential for spread of the hybrid microorganisms of the present invention to plants other than those with which they are intended to enter an endosymbiotic relationship. Many plant infecting microorganisms and their methods of interaction with plant hosts are described in the scientific literature. Particular reference in this regard is made to M.S. Mount et al., Phytopathogenic Prokaryotes, Volumes I and II (1982), which is specifically incorporated herein by reference. In addition, xylem-limited bacteria, such as the bacterium that causes Pierce's disease and that which inhabits C-4 grasses such as bermuda grass, will be useful in the practice of this invention. Such bacteria are described in Wells, et al., Phytopathology 71:1156-1161 (1981) and Raju, et al., Am. J. Enol. Vitic. 31:17-21 (1980) which are incorporated herein by reference.

Many pathogenic microorganisms are known to form strains essentially undifferentiable from each other except for their preference for particular plant hosts. Such host-specific pathogen strains are known as pathovars for the plant host involved. It is envisioned that the present invention will be particularly useful with pathovars for the particular host under consideration, including pathovars of the genera Agrobacterium, particularly Agrobacterium tumefaciens; Erwinia, particularly Erwinia stewartii and Erwinia cartovora; Pesudomonas, particularly Pseudomonas solanacearum and Pseudomonas syringae; Xanthomonas, particularly Xanthomonas campestris; and similar bacteria.

Particularly preferred non-pathogenic endosymbionts include species of the genus Azospirillum, particularly Azospirillum lipoferum and Azospirillum brasilense, the genus Acremonium, particularly Acremonium typhinum and Acremonium coenophialum, and the genus Balansia. Infecting microorganisms envisioned as suitable for use in accordance with the present invention include those listed in Table II.

Table II. Plant-Infecting Microorganisms

| Type | Genus | Species |
|------|-------|---------|
| Gram-Negative bacteria aerobic rods and doddi | Pseudomonas | agarici, andropogonis, avenae, caricapapaye, caryophylli, cichorii, cissicola, gladioli, glumae, marginalis, pseudoalcaligenes subsp. citrulli, rubrilineans, rubrisubalbicans, solanacearum, syringae, tolaasii, viridiflava |
| | Xanthomonas | albilineans, ampelina, axonopodis, campestris. fragariae |
| | Agrobacterium | rhizogenes, rubi, tumefaciens |
| Facultatively anaerobic rods | Erwinia | amylovora, ananas, cancerogena, carnegieana, carotovora subsup. atroseptica, carotovora subsp. carotovora, chrysanthemi, cypripedii, dissolvens, herbicola, mallotivora, milletiae, nigrifluens, nimipressuralis, quercina, rhapontici, rubrifaciens, salicis, stewartii, tracheiphila, uredovora |
| Gram-negative bacteria | Azospirillum | lipoferum, brasilense |
| Gram-positive bacteria Actinomycetes and related organisms | Corynebacterium | betae, beticola, fascians, flaccumfaciens, ilicis, insidiosum, michiganense, nebraskense, oortii, poinsettiae, sepedonicum |
| | Clavibacter | xyli |
| | Streptomyces | ipomoea, scabies |
| Fungi | Acremonium | typhinum, coenophialum |

Pathovars of Agrobacterium tumefciens are particularly preferred for formation of stable hybrids capable of producing agricultural chemicals and of entering into endosymbiotic relationships with dictyledonous plant hosts. Agrobacterium tumefaciens has a well identified interaction with the plant during the initial phases of infection in that it binds to plant cell tissue. The ability of Agrobacterium tumefaciens to bind to plant cell tissue in vitro has been demonstrated and has been shown to parallel the host range for tumor formation by Agorbacterium tumefaciens in vivo. See A.G. Matthysse, et al., "Plant Cell Range for Attachment of Agrobacterium tumefaciens to Tissue Culture Cells," Physiological Plant Pathology, 21:381-387 (1982); A.G. Matthysse, et al., "Binding of Agrobacterium tumefaciens to Carrot Protoplast," Physiological Plant Pathology, 20:27-33 (1982), both of which are specifically incorporated herein by reference.

Agrobacterium tumefaciens is the preferred infecting microorganisms for use in forming stable hybrids capable of producing agricultural chemicals and of entering into endosymbiotic relationships with sonanaceous plants, such as potatoes, tomatoes, tobacco, eggplant and pepper; brassicaceous plants, such as cauliflower, broccoli, cabbage, kale and kohlrabi; vegetables, such as carrot and parsley and other agriculturally grown plants, such as sugar beets, cotton, fruit trees, berry plants and grapes. Other infecting microorganisms suitable for use in accordance with the present invention to produce agricultural-chemical-producing microorganisms capable of entering into endosymbiotic relationships with dictyledonous plants include Erwinia carotovora, Pseudomonas solanacearum, Pseudomonas syringae and Xanthomonas campestris.

Infecting microorganisms such as Pseudomonas syringae and Xanthomonas campestris are envisioned as being particularly useful in the formation of stable hybrid microorganisms capable of producing agricultural chemicals and of entering into endosymbiotic relationships with monocotyledonous cereal crops, such as wheat, barley, rye, rice and oats, and also with grasses, such as brome grass, blue grass, tall fescue grass and bermuda grass.

Clavibacter species are useful as the infecting microorganism for such monocots as grasses, wheat, corn, and sorghum.

Strains of Clavibacter xyli subsp. cynodontis or subsp. xyli are particularly preferred as the infecting microorganism useful in forming stable hybrid microorganisms capable of producing agricultural chemicals and of entering into endosymbiotic relationships with monocotyledonous plants, such as tropical grasses, such as sugar cane, corn, millet and sorghum, and small grains such as rice. Strains of Pseudomonas syringae and Xanthomonas campestris are also envisioned as useful in these applications.

The above-identified strains of Azospirillum are envisioned as being useful in monocotyledonous plants, including tropical and temperate grasses, such as sugar cane and wheat, among others.

Species of the genus Balansia and the genus Acremonium, particularly Acremonium typhinum and Acremonium coenophialum, are useful as the infecting microorganism for grasses and forages, such as tall fescue, fine fescue, and ryegrass.

Strepotmyces species are useful as the infecting microorganism of root and tuber crops, such as sweet potatoe, white potato, sugar and other beets, and radishes.

It is to be understood that the selection of a particular plant-infecting microorganism and a particular agricultural-chemical-producing microorganism for use in accordance with the present invention will be within the capabilities of one having ordinary skill in the art through the exercise of routine experimentation in light of the teachings contained herein and inferences logically to be drawn from the disclosure of the present invention. The characteristics of microorganisms contemplated as useful as either the agricultural-chemical-producing microorganism or the infecting microorganism in processes of the present invention may be determined by routine experimentation or by reference to standard compendia, such as Bergey's Manual of Determinative Bacteriology, and the like.

For use in the present invention, it is preferred that mutants of the desired infecting microorganism be selected which have one or more selectable traps in addition to the ability to infect the plant host under consideration. Specifically, mutants of the infecting microorganism should be selected which have traits which are selectable in vitro. Such traits include antibiotic resistance, the need for specific nutritional supplementation (auxotrophism), resistance to toxins such as heavy metals, combinations thereof, and the like, which will allow rapid in vitro screening of the rhybrid microorganisms to identify those containing genetic material from the mutant infecting microorganism. In the absence of a selectable trait, physical means can be used to kill one or both parents but which will allow hybrids to survive. Such physical means include untraviolet light and heat.

Antibiotic resistance is the preferred selectable trait, and it is particularly preferred that the selected infecting mutant microorganism have resistance to at least two antibiotics. Dual antibiotic resistance, or redundancy in other selectable traits, ensures that the initial screening of the hybrids will identify only those having genetic material from both the agricultural-chemical-producing microorganism and the selected infecting mutant microorganism and reduces the chances of the survival or formation of mutant antibiotic resistant strains of the agricultural-chemical-producing microorganism. This is of particular value with fusion hybrids and is of little or no significance if cloned genes are employed. For example, in formation of agricultural-chemical-producing hybrids capable of producing fixed nitrogen and of entering into endosymbiotic relationships with monocyledonous plants, strains of Erwinia stewartii which have resistance to both streptomycin and tetracycline are preferred. In the formation of hybrids capable of producing fixed nitrogen and of entering into endosymbiotic relationships with dicotyledonous plants, strains of Agrobacterium tumefaciens which are both streptomycin resistant and kanamycin resistant are preferred. Such mutant strains of plant infecting microorganisms are available from microorganism depositories such as the American Type Culture Collection in Rockville, Maryland, or may be generated from the wild type by techniques well known in the art.

Where hybrids of the agricultural-chemcial-producing microorganism are not to be screened initially for the ability to produce the agricultural chemical in question, the agricultural-chemical-producing-microorganism may be a mutant with one or more additional selectable traits, preferably in vitro selectable traits, such as those mentioned above with respect to the infecting microorganism. Thus, for example, if the agricultural chemical of interest is an insecticide, nematocide or the like, the agricultural-chemical-producing microorganism may be a mutant which has selectable antibiotic resistance or sensitivity or a selectable need for specific nutritional supplementation. Obviously, these traits should not be identical to the selectable traits of the infecting microorganism since the initial screen would otherwise be unable to eliminate non-hybrid organisms. While the presence in the hybrids of such additional selectable traits from the agricultural-chemical-producing capability in the products of the initial screening procedure, it sufficiently enriches the population with true hybrid organisms with the agricultural-chemical-producing capability in subsequent screening.

In a particularly preferred embodiment, the hybrids of the present invention are formed by protoplast or spheroplast fusion of bacteria following the outline of techniques generally employed in the prior art. Known protoplast and spheroplast fusion techniques are described in D.A. Hopwood, "Genetic Studies With Bacterial Protoplast," Ann. Rev. Microbiol, 35:237-72 (1981), and in R .L. Weiss, J. Bacteriol., 128:668-70 (1976), both of which are specifically incorporated herein by reference in their entirety. In this embodiment, it is preferred, although not necessary, that the infecting bacterium and the agricultural-chemical-producing bacterium employed exhibit the same response to gram stain.

In general, the fusion procedure involves the removal of the cell wall from both the agricultural-chemical-producing bacteria and the infecting bacteria, fusion of the infecting bateria and agricultural-chemical-producing bacteria cells in a fusion inducing medium, such as polyethylene glycol, and regeneration of the cell wall about the fusion hybrids containing genetic material from both the infecting bacterium and the agricultural-chemical-producing bacterium. Fusion and regeneration of the cell wall are conducted at low temperatures so that rates of expressible genetic recombination are favored in relation to rates of enzymatic destruction of genetic material in the newly formed hybrids.

Following initial formation of fusion hybrids, the genetic make-up of the bacterial pouplation is relatively unstable for a period of two or three days, during which much genetic recombination apparently occurs. During this time preiod, those stable fusion hybrids capable both of manifesting the one or more selectable traits associated with the agricultural-chemical-producing bacteria and of manifesting the one or more selectable traits associated with the infecting bacterium are selected. It is particularly preferred that the agricultural-chemical-producing trait also be selected during this step. For example, fusion products of Azotobacter vinelandii and Erwinia stewartii formed from strains of Erwinia stewartii which are streptomycin and tetracyline resistant are grown in a nitrogen-free medium containing both streptomycin and tetracyclin. Thus, only those organisms containing both the genetic material associated with nitrogen fixation from Azotobacter vinelandii and the genetic material associated with streptomycin and tetracycline resistance from Erwinia stewartii will survive the two to three day incubation period.

The surviving fusion hybrids manifesting both the selectable traits associated with the agricultural-chemical-producing bacterium and the selectable traits associated with the infecting bacterium may then be screened for their ability to interact with plant tissue in the manner in which the infecting bacterium interacts with plant tissue during the intial phase of infection of the plant host. As noted above, the intitial phase of infection associated with Agrobcterium tumefaciens is binding to plant tissue cells may be determined in vitro by techniques hitherto described in the above-cited literature and by techniques described in the following examples.

Other infecting bacteria, such as Erwinia stewartii, initially interact with plant tissue following infection by spreading throughout the vascular system of the plant without being detected and destroyed by the plant's disease-response system. This character is assumed in the literature to be due to an extracellular polysaccharide produced by the infecting bacterium which permits it to elude the plant's normal defensive reaction. The ability of the fusion hybrids to spread throughout the vascular system of the plant in this manner may be determined in any convenient manner. One preferred screening technique involves the infection of seedlings of the host plant at a particular site in the plant. After a period of time, e.g., four days, the plant may then be dissected into a plurality of transverse sections displaced along the longtitudinal axis of the plant. Bacterial cultures may be regenerated from the bacteria contained in each section. The bacteria-containing sections farthest removed from the situs of initial infection will contain those fusion hybrids best able to disperse throughout the vascular system of the plant, thereby allowing selection of hybrids having this ability.

It is to be understood that other techniques for screening for this initial interaction with plant tissue may be envisioned by those skilled in the art in light of the teachings contained herein. It is to be further understood that other mechanisms of initial interaction between particular infecting bacteria and particular plant hosts may be quantified and screened for in ways which will be obvious to those skilled in the art in light of the teachings contained herein and know interactions between infecting bacteria and their plant hosts.

The ability to spread throughout the plant's vascular system is a desirable property even for fusion hybrids formed from infecting bacteria whose initial interaction with plant cell tissue is by some other mechanism, e.g., cell binding. Accordingly, it is preferred to screen further those fusion hybrids found to posses both the ability to produce agricultural chemical and the ability to interact with plant cell tissue in the manner in which the infecting bacterium interacts with the plant host during the initial phases of infection to select a subgroup of hybrids for further screening according to the present invention which are also capable of dispersing quickly throughout the vascular system of the plant.

Moreover, in practicing this embodiment of the present invention, it is preferred that the fusion hybrids found to have both the ability to produce agricultural chemicals and the ability to interact with plant cell tissue in the manner in which the infecting bacterium interacts with plant cell tissue during the initial phases of infection be screened for these capabilities two or more times. Such repeated screening tends to assure stability of the fusion hybrid strain and also allows selection for further screening of a manageable number of fusion hybrids having the greatest capabilities in these two areas.

The stable fusion hybrids manifesting both the selectable traits of agricultural-chemical-producing bacterium and plant-tissue-interaction capabilities, as above described, may then be grown as individual colonies, in the case of the agricultural-chemical-producing bacterium that fix nitrogen, preferably on nitrogen-free media. Each of the resultant cultures may then be applied in an appropriate manner, e.g., by injection, to seedlings of the plant host in question. After an appropriate incubation period, e.g., two or three weeks, those fusion hybrids which do not create any visible manifestation of a disease in the host plant seedlings may be selected for further screening. Experience has shown that screening of 25 to 30 of the best fusion hybrids is normally sufficient to provide 5 to 7 fusion hybrids which do not manifest visible disease symptoms, such as any associated with the original infecting bacterium, in the host plant in question.

Preferably, those stable fusion hybrids which manifest the widest area of spread throughout the vascular system of the plant, the clearest freedom from symptoms of disease in the host plant, and, in the case of agricultural-chemical-producing bacteria that fix nitrogen, the most vigorous growth in nitrogen-free media, are selected for further screening.

Some of the fusion products of the present invention have also been observed to form spores. Selection of endosymbiotic bacteria formed in accordance with the present invention which form spores facilitates application and survival of the bacteria when applied to plant host since the spores are generally more resistant to stress than the growing bacterial cells.

In another prefered embodiment, the agricultural-chemical-producing microorganisms of the present invention capable of entering into endosymbiotic relationships with a plant host are formed by the steps comprising:

(A) in any convenient order:
   (1) identifying the plant host; and
   (2) identifying an infecting microorganism which infects the plant host;
(B) preparing a vector capable of being transferred into and replicating in the infecting microorganism;
(C) preparing an expression module capable of directing the production of an agricultural chemical by the infecting microorganism;
(D) placing the expression module in the vector to create an expression vector capable of being transferred into and replicating in the infecting microorganism, the expression vector being capable of directing the production of the agricultural chemical in the infecting microorganism;
(E) transforming the infecting microorganism with the expression vector to produce hybrid microorganisms;
(F) selecting for the hybrid microorganisms;
(G) selecting serially from the selected hybrid microoganisms and in any convenient order:

(1) a subgroup comprising those hybrid microorganisms which manifest the ability to interact with plant tissue in the manner in which the infecting microorganism interacts with plant tissue during the initial phase of infection in the plant host;

(2) a subgroup comprising those hybrid microorganisms which, upon application to that host, do not create manifestations of disease; and

(3) a subgroup comprising those hybrid microorganisms having the ability to produce the agricultural chemical if not previously selected for; and

(H) selecting hybrid agricultural-chemical-producing microorganisms capable of entering into endosymbiotic relationships with a plant host by selecting from the products of the last performed step of steps (G)(1) to (G)(3) those hybrid microoganisms capable of improving the performance of the plant host under conditions wherein the performance would be improved by direct application of the agricultural chemical or the agricultural-chemical-producing microorganism to the plant host.

As used in the steps above, the term "selecting" means any intervention or combination of interventions such that the desired microorganism can be recognized either by its ability to survive or by its unique properties.

A vector capable of being transferred into and replicating in an infecting microorganism may be prepared by one skilled in the art in view of the teachings of the present invention. As used herein, the term "prepared" includes obtaining existing vectors known to have the desired properties.

An expression module is prepared using techniques known in the art. As used herein, an "expression module" is a DNA sequence capable of directing the production of a product by a cell, in this case an agricultural chemical by the infecting microorganism. It comprises a portable DNA sequence containing the structural gene or genes for the production of an agricultural chemical and transcription and translation control elements, the control elements being operable in the infecting microorganism. The expression module may also contain a DNA sequence that codes for a selectable trait in the infecting microorganism. That sequence may be controlled by the transcription and translation control elements previously mentioned or it may have its own transcript control elements that are operable in the infecting microorganism. It is preferred that the selectable trait be antibiotic resistance.

As used herein, the term "portable DNA sequence" is intended to refer either to a synthetically produced nucleotide sequence or to a restriction fragment of a naturally occurring DNA sequence. This sequence is obtained from the previously discussed agricultural-chemical-producing microorganisms. The methods of obtaining such sequence will be apparent to those of ordinary skill in the art in view of the teachings of the present invention and methods known in the art. Examples of such methods may be found in Maniatis, T., et al., Molecular Cloning, A Laboratory Manual (Cold spring Harbor Laboratory 1982), which is specifically incorporated herein by reference in its entirety.

One preferred way of making an expression module is as follows. A portable DNA sequence containing the structural gene or genes for the agricultural chemical is prepared. The portable sequence is then cloned into a vector containing transcription and translation control elements for the portable DNA sequence. These control elements are operable in the infecting microorganism. If the portable DNA sequence and the control elements are properly aligned by techniques known to those skilled in the art, an expression module is created in this vector. The expression module is capable of directing the infecting microorganism to produce the agricultural chemical. The module is then recovered by techniques known in the art.

There are alternative ways of making an expression module. For example, some or all of the control elements may be attached to the portable DNA sequence prior to its being cloned into the vector, in which case the vector need not contain those elements.

The transcription and translation control elements, as discussed herein, include at least one promoter, at least one ribosome binding site, at least one translation initation condon, and at least one translation termination codon. These elements may also include stability enhancing sequences and any other sequence necessary or preferred for appropriate transcription and subsequent translation of the vector DNA. These elements can include synthetic DNA, portions of natural DNA sequences, or products of in vitro mutagenesis. The particular control elements that are chosen for incorporation are chosen by criteria that are frequently empirically determined. That is, different configuration of control elements for example, the ribosome binding site and its flanking sequences, are tested for their effect upon expression directly until a successful configuration is evident. Such empirical determination involves techniques known to those skilled in the art.

After the expression module has been prepared, it is placed in the vector that is capable of being transferred into and replicating in the infecting microorganism, creating an expression vector. The expression vector is capable of being transferred into the replicating in the infecting microorganisms and capable of directing the production of the agricultural chemical by that microorganism.

This vector is transferred into the infecting microorganisms to produce hybrid microorganisms by techniques known to those of ordinary skill in the art in view of the teachings of the present invention. Not all of the infecting microorganisms will be transformed by the vector. Therefore, it will be necessary to select for the hybrid microorganisms. Such selection techniques will be apparent to those of ordinary skill in the art in light of the teachings of the present invention. One of the preferred selection techniques involves incorporating into the expression vector a DNA sequence, with any necessary transcription and translation control elements, that codes for a selectable trait in the infecting microorganism, if such a sequence is not already in the expression module. A preferred selectable trait is antibiotic resistance.

Once the hybrid microorganisms have been selected, it is necessary to select for those that are hybrid agricultural-chemical-producing microorganisms capable of entering into endosymbiotic relationships with a plant host. This is done by following the steps of (G)(1)-(G)(3) and (H) above as desired herein.

In a particularly preferred embodiment, the agricultural-chemical-producing DNA sequence is cloned into an integration vector, which is capable of integrating into the genome of the infecting microorganism. Use of such an integration vector prevents the transmission of the cloned DNA sequence to other microorganisms by plasmid transmission.

In particular, this method of producing hybrid agricultural-chemical-producing microorganisms capable of entering into endosymbiotic relationships with a plant host comprises:

(A) in any convenient order:

(1) identifying an infecting microorganism which infects the plant host;

(2) identifying an infecting microorganism which infects the plant host;

(B) preparing an integration vector containing an integration sequence and capable of integrating into the genome of the infecting microorganism;

(C) preparing an expression module capable of directing the production of an agricultural chemical by the infecting microorganism;

(D) placing the expression module within the integration sequence of the integration vector, thereby producing a modified integration vector capable of integrating into the genome of the infecting microorganism and directing the production of the agricultural chemical by the infecting microorganism;

(E) transforming the infecting microorganism with the modified integration vector to produce hybrid microorganisms;

(F) selecting for the hybrid microorganisms;

(G) selecting serially from the selected hybrid microorganisms and in any convenient order;

(1) a subgroup comprising those hybrid microorganisms which manifest the ability to interact with plant tissue in the manner in which the infecting microorganism interacts with plant tissue during the initial phase of infection in the plant host;

(2) a subgroup comprising those hybrid microorganisms which, upon application to the plant host, do not create manifestations of disease; and

(3) a subgroup comprising those hybrid microorganisms having the ability to produce the agricultural chemical, if not previously selected for; and

(H) selecting hybrid agricultural-chemical-producing micro-organisms capable of entering into endosymbiotic relationships with a plant host by selecting from the products of the last performed steps of steps (G)(1) to (G)(3) those hybrid microorganisms capable of improving the performance of the plant host under conditions wherein the performance would be improved by direct application of the agricultural chemical or the agricultural-chemical-producing microorganism to the plant host.

As used in the steps above, the term "selecting" means any intervention or combination of interventions such that the desired microorganism can be recognized either by its ability to survive or by its unique properties.

The integration vector can be prepared by techniques known in the art. It must have a DNA sequence homologous to a natural DNA sequence from the organism whose genome is the integration target. This sequence is known as the integration sequence. In a particularly preferred embodiment, the integration vector is pCG300, which has been deposited in the American Cultural Collection, 12301 Parklawn Drive, Rockville, MD 20852, U.S.A. and assigned Accession No. 53329.

The expression module may be prepared as previously described herein or by other techniques known in the art. It should be noted that a selectable trait need not be in the expression module. It may be elsewhere in the integration vector, but it is preferably within the present invention, it is preferred that the expression module be prepared by:

1) preparing a vector containing a promoter that is operable in the infecting microorganism;

2) placing into the vector a portable DNA sequence containing the structural gene or genes for the production of the agricultural chemical and containing transcription and translation control elements other than the promoter, which are operable in the infecting microorganism, to produce an expression vector containing an expression module, wherein the expression module is operable in the infecting microorganism; and

3) recovering the expression module from the expression vector.

As previously mentioned, the control elements, and the portable DNA sequence will need to be properly positioned by techniques known in the art in order to create an operable expression module.

The vector prepared by the first step in the preceding paragraph is often referred to as a promoter vector. Such a promoter vector is useful for moving a promoter from the infecting microorganism for the creation of the expression module. A particularly preferred promoter vector is the plasmid pCG6, which has been deposited in the American Type Culture Collection, 12301 Parklawn Drive, Rockville, MD 20852, U.S.A. and given the Accession No. 53328.

As previously mentioned, there are alternative ways to make expression modules useful in the practice of the present invention that will be apparent to those skilled in the art in view of the teachings herein. In addition, the transcription and translation control elements may be obtained in several ways. They may be associated with the structural gene or genes in the portable DNA sequence, or they may also be created synthetically or by site-specific mutagenesis.

When the desired expression module is produced, it is removed and cloned into the integration vector, preferably within the integration sequence. The objective is that through double cross-over events flanking the expression module, only that portion of the integration vector into the genome of the infecting microorganism. In the event that the expression module has been cloned outside of the integration sequence, the entire integration vector may be integrated. In either event, the modified integration vector is capable of directing the production of the agricultural chemical by the infecting microorganism.

The modified integration vector is then used to transform the infecting microorganism to produce hybrid microorganisms. Since only some of the infecting microorganisms will be transformed, it is necessary to select for such hybrid microorganisms. There are many such selection techniques known to those of ordinary skill in the art. A preferred selection technique is to select for a marker or a trait such as antibiotic resistance in the hybrid microorganism.

Once they have been selected, the subgroups of hybrid microorganisms specified in steps (G)(1)-(G)(3) are selected for serially as described herein. Then, hybrid agricultural-chemical-producing microorganisms capable of entering into endosymbiotic relationships for the plant host are selected by selecting those hybrid microorganisms capable of improving the performance of the plant host under conditions wherein the performance would be improved by direct application of agricultural chemical or the agricultural-chemical-producing microorganism to the plant as described herein.

For the embodiment of the present invention involving the use of recombinant DNA techniques, the plant host with which the hybrid microorganisms may establish endosymbiotic relationships, the infecting microorganisms, and the microorganisms that are the source of the agricultural chemical producing gene or gene include all of those mentioned previously, herein. However, it is preferred that the infecting microorganism and the microbial source of the agricultural chemical producing gene or genes are bacteria. Most preferably, the infecting bacterium is a species of the genus Corynebacterium or the genus Clavibacter. Clavibacteria xyli subsp. cynodantis and Clavibacter xyli subsp. xyli are expecially preferred. When the infecting bacterium is a species of the genus Clavibacter, it is preferred that the plant host be of the Gramineae family. In this case, particularly preferred hosts include bermuda grass, sugar cane, sorghum, and corn.

A preferred agricultural chemical is the delta endotoxin of Bacillus thuringiensis. Various Bacillus thuringiensis delta-endotoxin genes have been described, for example, in Klier, A., et al., The EMBO Journal, 7:791-799 (1982) and Schnepf, H.E., et al., Proc. Natl. Acad. Sci, USA, 5: 2893-2897 (1981), both of which are specifically incorporated herein by reference in their entirety. A preferred source of the gene for the delta toxin is the M13 vector mBTK65, deposited in the American Type Culture Collection, 12301 Parklawn Drive, Rockville, Maryland 20852, U.S.A. under Accession No. 53326. It contains a truncated B. thuringiensis delta endotoxin gene fused to a kanamycin resistance gene in such a manner that both insecticidal activity and kanamycin resistance are expressed.

If not conducted in the initial screening of hybrid microorganisms, the hybrid organisms must, sooner or later, be screened directly for the ability to produce the agricultural chemical of interest. Preferably, the screening should be conducted at the earliest practical point in the process, which will be dictated by the number of hybrid microorganisms surviving at any particular stage of the process and the power of the technique used to screen for production of the agricultural chemical of interest to identify agricultural-chemical-producing hybrids out of a mixed inoculum.

It is to be understood that the screening for a particular agricultural-chemical-producing trait for use in accordance with the present invention will be within the capabilities of one having ordinary skill in the art through the exercise of routing inferences logically to be drawn from the disclosure of the present invention. The techniques contemplated as useful in screening for ability to produce the agricultural chemical may be determined by reference to standard compendia.

In general, these screening procedures will fall into one of four categories. These are (1) survival screens, where the agricultural chemical being produced is essential to survival of the hybrid and is not provided by the growth media; (2) analytical chemistry screens for the presence of the agricultural chemical being produced; (3) biological screens for the manifestations of biological activity associated with the agricultural chemical or chemicals being produced; and (4) immunoassay screens whereby organisms or groups of organisms producing a particular agricultural chemical are identified by interaction with an antibody which is specific to the chemical in question. The suitability of any particular screening technique or combination of techniques will be dictated by the agricultural chemical in question. Thus, survival screens are particularly useful in identifying hybrid microorganisms capable of producing their own fixed nitrogen by survival on nitrogen-deficient media. Biological screens are particularly useful in indentifying hybrid microorganisms capable of producing antibaterial or antifungal agents. A particularly preferred screening technique of this sort involves layering a culture of organisms susceptible to the antibiotic produced by the hybrid microorganism over a mixed culture of hybrids and identifying and removing for further screening hybrids or groups of hybrids producing the antibiotic from those areas where the susceptible organism cannot grow. Immunoassay techniques are particularly appropriate where the agricultural chemical of interest is a large molecule, such as polypeptide. In any event, biological screens for the known effects of the agricultural chemical in question can be conducted using discrete cultures of hybrid microorganisms once the number of such cultures has been reduced to a manageable level, e.g., $10^2$-$10^3$ cultures, by preliminary screens for other selectable traits associated with the agricultural-chemical-producing microorganism. Thus, hybrids producing insecticidal compounds, for example, could not be identified by the inability of susceptible insects to survive on a culture of the hybrid. Such screening has the disadvantage of requiring screening of a larger number of colonies and requiring a protracted period of time to manifest results but is nonetheless capable of identifying hybrids with the ability to produce the agricultural chemical of interest.

It is to be understood that, with appropriate changes in the screening procedure, hybrids capable of fixing nitrogen anaerobically, as is done in Rhizobium, or in the presence of very small amounts of oxygen, as is done by microaerophilic bacteria, can be prepared in accordance with the present invention. Specifically, oxygen must be minimized or removed in the nitrogen free growth medium during screening for nitrogen-fixing ability.

If such anaerobic nitrogen-fixing hybrids are to enter into endosymbiotic relationships with plant host, the hybrid must also contain genetic material, such as that contained in species of Rhizobium, which will cause the plant host to create an oxygen free or low oxygen environment not normally found in crop plants. For this reason utilization of aerobic nitrogen-fixing bacteria is preferred.

In order to determine which of the final group of hybrid microorganisms is capable of entering into endosymbiotic relationships with the host plant whereby agricultural chemcials produced by the hybrid may be utilized by the plant as a supplement to agricultural chemicals otherwise available, it is normally necessary to conduct preliminary yield gain screening or the like. For nitrogen-fixed hybrid bacteria produced by cell fusion, for example, this is accomplished by growing the host plant in an environment deficient in the agricultural chemical e.g., fixed nitrogen, and comparing the dry weight after an appropriate period of growth, e.g., six weeks, with the dry weight of similar plants in similar environment but which had been infected, as by injection, with the stable fusion hybrid bacteria selected by the foregoing method. In the case of agricultural-chemical-producing bacteria that fix nitrogen, experience has shown that testing of 5 to 7 of the best fusion hybrids will result in identification of 3 or 4 stable fusion hybrids capable of creating statistically significant yield gains in the host plant growing in nitrogen deficient soil due, it is believed, to the ability of the stable fusion hybrids to fix atmospheric nitrogen aerobically and to enter into endosymbiotic relationships with the host plant.

It is envisioned that the endosymbiotic agricultural-chemical-producing microorganism formed in accordance with the present invention may be further modified by natural or artificial genetic techniques to improve their performance as sources of agricultural chemicals for the plant host. Specifically, the microorganisms may be modified to reduce their resistance to cold temperatures, thereby preventing unintended proliferation of the hybrid microorganisms from year to year. Similarly, the endosymbiotic hybrid microorganisms formed in accordance with the present invention may be modified to enhance their stability to drought, disease or other physiological stress. In this regard, the stability of the hybrid under stress conditions, and the ability to minimize the likelihood of spontaneous or forced reversion to a pathogenic state, is desired in a commercial microbiological product. In addition, the hybrid microorganisms could be modified to excrete the agricultural chemical. Ideally, the microorganism formed in accordance with the present invention should be modified so that they cannot grow outside the plant host, as by the selection of mutants requiring nutritional supplementation specifically or nearly specifically provided by the plant host in question. Techniques for genetic manipulation and mutant selection for strains of, for example, Azotobacter are well known in the art and are envisioned as being suitable for effecting the above-described and similar genetic manipulations of the stable hybrids formed in accordance with the present invention.

The stable hybrid microorganisms having the ability to produce agricultural chemicals and of entering into an endosymbiotic relationship with a plant host formed in accordance with the present invention may be used to prepare agricultural products in many ways. For example, hybrids formed in accordance with the present invention may be injected into seedlings or used to form coated seed of the plant host involved by association of the hybrid with a biodegradable nutrient carrier material which is coated on the seed. Similarly, hybrids formed in accordance with the present invention may be used to form infected seed products by application of the microorganisms directly to the seed of host plants. Alternatively, an agricultural soil drench may be prepared by mixing microorganisms formed in accordance with present invention with water and other appropriate drench constituents for application to the leaves, stem and roots of growing host plants and surrounding soil by spraying or the like.

It is to be understood that application of the teachings of the present invention to a specific problem or environment will be within the capabilities of one having ordinary skill in the art in light of the teachings contained herein. Examples of the process within the scope of the present invention and the microbial products resulting therefrom appear in the following examples.

Example 1

### Preparation of a Fusion Hybrid Bacterium From Azotogacter vinelandii and Erwinia Stewarii Capable of Fixing Atmospheric Nitrogen Aerobically and of Entering Into an Endosymbiotic Relationship With Corn

Approximately $10^8$ bacteria of the species Azotobacter vinelandii known to be capable of fixing nitrogen aerobically were selected. Approximately $10^8$ bacteria of a strain of Erwinia stewartii mutants known to be resistant to both streptomycin and tetracycline were also selected. Fusion hybrids of these two types of bacteria were formed by a variant of the procedure of Weiss referred to above.

The bacteria growing in mid-log phase were treated with lysozyme at a concentration of 100 micrograms per milliliter and with DNA-ase at a concentration of 5 micrograms per milliliter in media containing EDTA at a concentration of 1 millimolar, having a pH of 7 and with 12% added sucrose. After 10 minutes, protoplasts and spheroplasts of the original bacteria were formed. The temperature was then lowered to 0°C and the protoplast-forming solution was washed out. The resulting protoplasts were placed in 12% sucrose solution at 0°C. The solution was then supplemented with 40% polyethylene glycol of molecular weight about 6,000 in order to induce protoplast and spheroplast fusion for a period of 10 to 15 minutes. The fused bacteria were spun down in a centrifuge and resuspended in 12% sucrose plus a complete growth medium for 4 hours at 0°C. The temperature was then raised to 30°C and was held there for 2 hours, after which the hybrid fusion products were transferred to complete growth medium containing no additional sucrose for 2 hours.

The hybrid fusion products were transferred and grown on Burke's nitrogen-free medium as described in Carlson, et al., "Forced Association Between higher Plant and Bacterial Cells in vitro," Nature, Vol. 22, No. 5482, p. 393-395 (1974). The medium was nitrogen-free and contained added streptomycin at a concentration of 100 micrograms per millimeter and tetracycline at a concentration of 50 micrograms per milliliter. Only those fusion products manifesting both the ability to fix atmospheric nitrogen aerobically and manifesting the streptomycin and tetracycline resistance associated with the Erwinia stewartii strain originally introduced survived on this medium after a growth period of about 3 days.

Samples of the mixed fusion hybrids from this culture were used to infect corn seedlings approximately 3 to 4 feet in height by injection between the first and second node of the stalk tissue. After 4 days the corn plants were dissected into transverse sections to recover bacteria that moved to the anterior (top) of the plant, since Erwinia stewartii interacts with plant tissue during the initial phase of infection by spreading throughout the vascular system of the plant without being recognized or destroyed by the plant's disease-response system. Those fusion hybrids with the greatest facility to spread throughout the vascular system of the corn plant were generally found between the fifth and sixth node.

The fusion hybrid bacteria found between the fifth and sixth node of the corn plant were cultured for 3 days on Burke's nitrogen-free medium as described by Carlson, et al., surpa. After 3 days, a mixed inoculant from this nitrogen-free culture was again used to infect corn seedlings approximately 3 to 4 feet in height by injection between the first and second node of the stalk tissue. After about 3 days the plant was transversely dissected as above described to recover bacteria that moved to the anterior of the plant Again, those fusion hybrids with the greatest facility to spread between the fifth and sixth node.

The fusion hybrid bacteria recovered from this portion of the corn plant were then grown as separate colonies on Burke's nitrogen-free medium. Twenty-five of the most vigorous colonies were selected, and each was used to infect one of 25 corn plants by injection. After 3 weeks, those colonies which did not result in any visible manifestation of disease in the infected corn plants were selected. From among these, seven colonies which manifested the most vigorous growth on nitrogen-feee media, the widest area of spread throughout the plant and least manifestation of disease symptoms were selected for preliminary yield testing and were identified as Fusion #1 through Fusion #7.

In the preliminary yield test, flats (30cm x 46cm) containing 15cm of washed sterile sand over 10cm of Perlite were sown with 24 corn seeds at a uniform spacing. Flats were maintained in the greenhouse (at approximately 22-27°C) and were watered twice daily with deionized water. Starting at 10 days (so as to exhaust the seed nitrogen) the seedlings were watered three times a week with a nutrient solution. One liter of solution was a modified Long Ashton mix that contained nitrate ion at a concentration of 6 micromolar as its sole nitrogen. The Long Ashton mix was also modified to contain nitrate ion at concentrations of 6 and 0 millimolar and used in comparative runs A and C below, respectively. Experiments were run for six weeks, at which time the aerial portions of the plants were harvested and dried for containing infected plants, the bacterial strains were introduced by injection at 10 days (at the same time as fertilization was initiated). Each analysis was done in triplicate (three flats of 24 plants per treatment) and that data were subjected to statistical analysis. Seven different bacterial fusions were selected by multiple screens between Erwinia stewartii and Azotobacter vinelandii (Fusion #1 through #7) as above described and used in this work. Actual dry weight yield data per flat (average of the three flats) is as follows:

| Inoculation | NO$_3$ Level | Average Flat Dry Weight (grams) | Percentage Increase Over Control |
|---|---|---|---|
| A. None | 6 millimolar | 116.8 | - |
| B. None (Control Value) | 6 micromolar | 34.8 | - |
| C. None | None | 19.2 | - |
| Fusion #1 | 6 micromolar | 44.8 | 29 |
| Fusion #2 | 6 micromolar | 39.6 | 14 |
| Fusion #3 | 6 micromolar | 72.4 | 108 |
| Fusion #4 | 6 micromolar | 33.2 | -5 |
| Fusion #5 | 6 micromolar | 54.8 | 57 |
| Fusion #6 | 6 micromolar | 25.2 | -28 |
| Fusion #7 | 6 micromolar | 56.8 | 63 |

Results with Fusion numbers 1, 3, 5 and 7 are statistically significant.

Uninoculated comparative run A contains the optimum level of nitrate in Long Ashton nutrient solution and is indicative of results to be expected with corn growing in well introgen-fertilized soil. Comparative run C, to which no nitrate was added, shows the yield which may be expected based only on residual nitrate contained in the seed of the corn. Comparative run B, which represents the control value containing nitrogen at levels of about 6 micromolar, is indicative of growth to be expected in poorly nitrogen-fertilized soil. The reduction in dry weight associated with fusion product number 6 is apparently due to non-visible pathogenic effects, even though none of the plants manifested visual symptoms of disease. The results of fusion product number 4 are not statistically significant. The results of fusion products 1, 3, 5 and 7 show that the process of the present invention was able to produce at least four stable fusion hybrid products capable of fixing atmospheric nitrogen aerobically and capable of entering into endosymbiotic relationships with corn whereby corn growing under nitrogen stressed conditions in conjunction with the fusion products of the present invention produced yields of from 29% to 108% greater than a control without the bacteria formed in accordance with the present invention.

Example 2

## Preparation of a Fusion Hybrid Bacterium from Azotobacter vinelandii and Agrobacterium tumefaciens Capable of Fixing Atmospheric Nitrogen Aerobically and of Entering Into an Endosymbiotic Relationship with Sugar Beets

Approximately $10^8$ bacteria of the species Azotobacter vinelandii were selected. Approximately $10^8$ bacteria of strain of Agrobacterium tumefaciens mutants which are resistant to both streptomycin and kanamycin were selected. Protoplast fusion was effected between these bacteria as in Example 1. The fused protoplasts were transferred to Burke's nitrogen-free medium as described in Example 1 supplemented with streptomycin at a concentration of 100 micrograms per milliliter and kanamycin at a concentration of 50 micrograms per milliliter. After 3 days, only those stable fusion hybrids capable of both fixing atomspheric nitrogen aerobically and possessing the genetic material from the pathogenic bacteria associated with streptomycin and kanamycin resistance survived.

A mixed inoculum from this culture was introduced into a suspension culture of carrot tissue cells in the manner described by A.G. Matthysse, et al., Physiological Plant Pathology, 20, 27-33 and 21, 381-387 (1902), which is specifically incorporated herein by reference in its entirety. After about 3 hours, the carrot cells and those fusion hybrids capable of binding to carrot cells in a manner similar to the initial interaction of Agrobacterium tumefaciens with plant tissue cells during the initial phases of infection were separated from unbound bacteria by washing 3 times in sterile, distilled water. The carrot cells with adhering hybrid bacteria were macerated and washed again. The resulting suspension was spun down in a centrifuge and the resulting pellet, consisting of cell walls and adhering hybrid bacteria, was placed on Burke's nitrogen-free medium for 3 days.

The cell-binding method as above-described was repeated and the fusion hybrids thus selected were grown as separate colonies on nitrogen-free media. After 3 days of growth, 25 of the most vigorous colonies were selected.

Each colonies was used to infect of 25 sugar beet seedlings 8 to 10 inches high by insertion of a pin, which had been dipped into the colony, into the area of the hypocotyle between the root and the plant. Those hybrid colonies which did not form a gall or tumor in the sugar beet seedling after 3 weeks were selected for further screening.

The roots of each infected sugar beet plant which did not manifest any visible symptoms of disease after 3 weeks were then dissected into a number of transverse sections. The distance down the root of the plant to which the hybrid bacteria had spread was noted. Five of the 25 stable fusion hybrids were selected for preliminary yield testing based upon the vigorousness of their growth on nitrogen-free media, the absence of visible manifestations of disease associated with their injection onto the plant and the facility with which they were able to spread throughout the plant tissue. These five fusion hybrids, designated Fusion #1 through Fusion #5 were subjected to preliminary yield testing as follows:

Flats (30cm x 46cm) containing 15cm of washed sterile sand over 10cm of Perlite were plated with 24 germinated sugar beet seeds at a uniform spacing. Flats were maintained in the greenhouse (at approximately 22-27°C) and were watered twice daily with deionized water. Starting at 10 days (so as to exhaust the seed nitrogen) the seedlings were watered three times a week with a nutrient solution. One liter of solution was applied and allowed to drain through. The Long Ashton mix was also modified to contain nitrate ion at concentrations of 6 and 0 millimolar and used in comparative runs A and C below, respectively.

Experiments were run for six weeks, at which time the plants were harvested and dried for one week at 95°C. Dry weight data was then collected. For flats containing infected plants, the bacterial strains were introduced by injection at 10 days (at the same time as fertilization was initiated). Each analysis was done in triplicate (three flats of 24 plants per treatment) and the data were subjected to statistical analysis. Five different bacterial fusions were selected by multiple screens between Agrobacterium tumefaciens and Azotobacter vinelandii as above described and used in this work. Actual dry weight yield data per flat (average of three flats) is as follows:

| Inoculation | NO$_3$ Level | Average Flat Dry Weight (grams) | Percentage Increase Over Control |
|---|---|---|---|
| A. None | 6 millimolar | 44.7 | - |
| B. None (Control Value) | 6 micromolar | 8.8 | - |
| C. None | None | 5.2 | - |
| Fusion #1 | 6 micromolar | 8.0 | -9 |
| Fusion #2 | 6 micromolar | 12.4 | 41 |
| Fusion #3 | 6 micromolar | 6.9 | -22 |
| Fusion #4 | 6 micromolar | 24.6 | 180 |
| Fusion #5 | 6 micromolar | 14.9 | 69 |

Results with Fusion numbers 2, 3, 4 and 5 are statistically significant.

Uninoculated comparative runs A, B, and C have the same significance as uninoculated comparative runs A, B, and C in Example 1 above. Fusion product No. 3 appeared to result in non-visible pathogenic effects, even though no visible symptoms of disease were observed on any of the plants. The preliminary yield data confirms that the process of the present invention was capable of producing at least three stable hybrid bacteria (Fusion #'s 2, 4 and 5) capable of fixing atmospheric nitrogen aerobically and of entering into an endosymbiotic relationship with sugar beets whereby sugar beets growing in nitrogen stressed conditions generated yields 41 to 180% in excess of those obtained with uninoculated sugar beets.

Example 3

Example 2 was repeated with the exception that the fusion products were applied to sugar beets receiving nitrogen in the form of nitrate at a concentration of 6 millimolar --that associated with the optimal nitrogen fertilization. No significant yield gain above that attained by uninoculated comparative run A was observed in the inoculated sugar beets. These results confirm that the yield gains associated with the fusion products in Example 2 were due to an endosymbiotic relationship between the fusion products and used by the plant host. Had the yield gains reported in Example 2 been the results of growth regulators or hormones provided by the fusion products, similar gains should have been observed in Example 3.

Further evidence of an endosymbiotic association with the host is provided by light microscopy using gram stain and form reisolation of the bacteria (with the expressions of the maintained antibiotic resistance markers) from infected plants. Moreover, the hybrid bacteria can be found throughout the plant. Their location is not limited to the site of infection. The number of hybrid bacteria in plant tissue was approximately $10^5$ per gram (dry weight) in a corn plant growing under low nitrate conditions.

Example 4

Production of a Fusion Hybrid Expressing Invertebrate Pesticide Activity for Use in Sweet Potatoes

The streptomycete S. ipomoea is a pathogen of Ipomoea species, which includes morning glories and sweet potatoes (Ipomoea batata). This organism invades both the fleshy root and fibrous roots of the latter, and is an endophyte candidate for fusion with an appropriate agrochemical producing bacterium. Such a partner is S. avermitilis, which produces a family of compounds called avermectins, which are effective against a variety of insects pathogens, such as nematodes, mites, and insects. Campbell, W.C. et al., Science 221: 823-828 (1983). Using the technique of protoplast fusion, hybrids of S. ipomoea and S. avermitilis were produced, which can associate with sweet potato tissue and produce avermectins.

Spores ($10^8$) of S. avermitilis strain SA-5 (nic) on deposit with the American Type Culture Collection, 12301 Parklawn Dr., Rockville, Maryland 20852 USA under Accession No. 53319 and S. ipomoea wild type SI-1 on deposit with the American Type Culture Collection, 12301 Parklawn Dr., Rockville, Maryland 20852 USA under Accession No. 55320 were inoculated into, respectively, 30 Ml of YSG 1 and YSG 2 broth (modified YSG 1 with 14% sucrose and 0.2% glycine). YSG 1 was prepared according to the method of Chater, K.F., et al., Cur. Top. Micro. Bio. Immunol. 96: 69-95 (1982), specifically incorporated herein by reference in its entirety. Both cultures were incubated at 30° with vigorous aeration (240 rpm) for 24 hours. Protoplasts were prepared from both strains and suspended in protoplast buffer med P as described in Hopwood, D.A., et al., Mol. Gen. Genet. 162: 307-317 (1978), specifically incorporated herein by reference in its entirety. SI-1 protoplasts were irradiated with UV light at 254 nm (Model UVGL-58 Mineralight Lamp, UVP Inc., San Gabriel, California) at 8cm from the source for 2 minutes to give 1% cell survival. UV-irradiated SI-1 protoplasts ($10^7$) were mixed with $10^7$ SA-5 protoplasts and sedimented by centrifugation at 6000 rpm (Sorvall SS-34 rotor) for 5 min at 4°C. The pelleted protoplasts were resuspended gently in 0.1 ml of med P, and 0.9 ml of 40% (wt/vol) PEG 8000 (Sigma) in med P was added following by gentle mixing. After incubation at 25°C for 3 minutes, the PEG-treated protoplasts were diluted 10-fold with med P, centrifuged and suspended in 2 ml of med P as previously. Samples (0.1 ml) of the fused protoplasts were plated at 30°C onto protoplast regeneration plates (RM16: yeast extract 0.2%, casamino acids 0.01%, sucrose 15%, glucose 1%, oatmeal 0.3%, L-proline 0.1%, $MgCl_2.2H_2O$ 1%, $Na_2SO_4$ 0.025%, agar 2%, trace element (Hopewood, D.A., op. cit.) 0.2%. After autoclaving the following sterile solutions were added: $CaCl_2$ 20 mM; $KH_2PO_4$ 5mM, MES (2-(N-Morpholino) ethane sulfonic acid) buffer (pH 6.5),25 mM). The regenerated colonies were replicaplated by cellulose acetate membrane filters (pore size 0.45um, diameter 82 mm, Micro Filtration System, Japan) onto minimal agar HMM plates (Hopwood D.A., Bacterial Rev., 31: 373-403 (1967)) to screen for prototrophs. The latter were subsequently patched onto YDC plates (yeast extract 0.4%, malt extract 1%, dextrose 0.4%, maltose 0.4%, agar 1.5%. After autoclaving, $MgSO_4$ (10 nM) and CaCl 6.25 mM were added. The plates were incubated for 7 days until the cells had sporulated, and those with SI-1 colony morphology and inability to produce melanin on YDC plates were further screened for avermectin activity by a toxicity test using freshly hatched brine shrimp. A 1 $mm^2$ piece of colony was placed in the well of a 96-well microtiter dish (nunc) and 0.01 ml of methanol added. Brine shrimp (ca. 10 per 0.1 ml) were added and the time of paralysis noted, and compared to controls. Strain SA-5 produced complete paralysis in 15 minutes at 22°C.

Among $10^5$ regenerated colonies, 270 showed SI colony morphology. Ten percent of the latter exhibited avermectin activity (Paralysis within one hour), and 6 of these positive clones were further inoculated onto sweet potato slices to assay for their ability to associate and grow in plant tissue Moyer, J.W. et al., Phytopathology, 74: 494-497 (1984). All 6 grew on sweet potato slices, and 2 of these recovered from the plant tissue retained avermectin activity.

Example 5

Endophyte Hybridization with Recombinant DNA

The bacterial species Clavibacter xyli subsp. Cynodontis (Cxc) and subsp. xyli (Cxx) are vascular inhibitors of bermuda grass and sugar cane, respectively, Davis, M.J. et al., Int. J. Syst. Bacteriol., 34: 107-117 (1984), which is specifically incorporated herein by reference in its entirety. They can grow in other commercially valuable crops such as sorghum. Liao, C.H., et al., Phytopathology, 71: 1303-1306 (1981), which is specifically incorporated herein by reference in its entirety. It has been discovered that Cxc and Cxx can also grow in field and sweet corn without pathogenic effect. The following example teaches how the organism Cxc can be modified to produce an insecticidal protein, the Delta endotoxin of Bacillus thuringienesis var. Kurstaki HD-73, which is active upon lepidopterous insect such as corn earworm (Heliothus zea) and European corn borer (Pyrausta nubilalia), which are pests of the field and sweet corn.

Preparation of Integration Vector

The genes for agricultural chemicals are preferably carried on an integration vector for Cxc to prevent transmission of cloned genes to other organisms by plasmid transmission. Integration vectors have the foreign gene sequences inserted into or adjacent to a natural sequence of DNA from the organism whose genome is the integration target. See Saunders, C.W. et al., J. Bacteriol. 157: 718-726 (1984). The integration vector is commonly propagated in a permissive host such as E. Coli or B. subtilis.

The manipulation of the foreign agricultural chemical gene sequences is carried out in E. Coli or B. subtilis using replication vectors for these hosts. When the desired gene configuration is produced, the configuration is removed and cloned into the Cxc integration vector adjacent to or within the Cxc cloned DNA segment, and the integration vector is propogated in the permissive host. Purified integration vector DNA is then used to transform the desired Cxc host, selecting for a marker such as drug resistance, which is also carried in the foreign gene configuration. Since the vector does not carry a replication origin for propagation in CXc, the vector must recombine with the Cxc chromosome in order to persist in Cxc. Recombination is facilitated by the Cxc homologous sequence on the integration vector. The stable drug-resistant transformants are then screened for the production of the agricultural chemical gene porducts, e.g. B. thuringenesis endotoxin.

Recombinant DNA Methods

The restriction enzymes, T3 DNA ligase and calf intestinal alkaline phosphatase, used in the following methods were purchased from New England BioLabs, Bethesda Research Laboratories, or Boehringer Mannheim. The reaction conditions were those recommended by manufacturer.

Isolation of TBIA DNA

A 3 litre culture of Cavibacter xyli subsp. cynodotis (TBIA, on deposit with the American Type Culture Collection, 12301 Parklawn Dr., Rockville, Maryland 20852 U.S.A. under Accession No. 33973) was grown aerobically in S8 medium (Davis, M.J., et al., Science 210: 1365-1367 (1980)) to mid-exponential stage at 28°C, at which point glucose and glycine were added to final concentrations of 2% and 0.1%, respectively. After growth for 17 hours, the cells were centrifuged, washed with lysis buffer (50 mM glucose, 25 mM Tris-Cl (pH 8.0), mM EDTA)) and resuspended in 2 ml of the same. The cells were then lysed by addition of guanidine hydrochloride and sarkosyl to final concentration of 7M and 4%, respectively, and Tris-Cl (pH 8.0) to 20 mM, aNd EDTA to 20mM. Sung, M.T., et al., Genetic Engineering in the Plant Sciences, N.J. Parapoulos (ed.), pgs. 39-62 (Praeger Scientific Press 1981), specifically incorporated herein by reference in its entirety. The final volume was 20 ml. After overnight incubation at 50°C, 4 ml of distilled water was added to the lysed suspension and the mixture spun at 12,000 rmp for 10 minutes at 20°C in the SS34 Beckman rotor. This was done to separate cell wall and membrane debris from the nucleic acids remaining in the supernatent. The supernatent fluid was mixed with ethidium bromide (final concentration equal to 600 ug/ml) and applied to a 2-step cesium chloride gradient which was centrifuged for 24 hr., 26000 rmp at 20°C in a Beckman SW27 rotor. The top step consisted of 4.42 M CsCl, 10 mM Tris-Cl (pH 8.0), 1 mM EDTA to a final density of 1.55; the bottom step consisted of 5.7M CsCl, 0.02M Tris-Cl (pH 8.0), to final density of 1.7. The DNA band was extracted and purified by standard techniques. Maniatis, T., et al., Molecular Cloning, A Laboratory Manual (Cold Spring Harbor Laboraotry 1982), specifically incorporated herein by reference in its entirety.

TBIA Chromosomal Fragment Isolation

TBIA chromosomal DNA was partially digested with the restriction enzyme, Sau3AI, resulting in DNA fragments ranging from approximately 1 kb to 25 kb (as shown by 0.7% agarose gel electrophoresis). DNA fragments approximating 10 kb were isolated by centrifuging the partial Sau3AI digest of TBIA DNA through a sucrose gradient. M.R. El-Gewely and R. B. Helling, Anal. Biochem. 102: 423-428 (1980).

Cloning of TBIA DNA Fragment

The cloning vector, pUC19 (New England Biolabs 1985-86 Catalogue, p. 90-91) was digested with the restriction enzyme, BamHI, to create a linear plasmid with cohesive ends. To prevent self-religation, the ends were treated with calf intestinal alkaline phosphatase. Maniatis, T., et al., op. cit. The Sau3AI digested TBIA fragments approiximating 10 kb (isolated by above procedure) were then ligated into the unique BamHI site of pUC19 using T4-DNA ligase. The concentrations of vector (pUC19) and insert (Sau3AI digested TBIA fragments) used to obtain the correct circular constructions were 0.0044 and 0.0156 ug per microliter of ligation mix, respectively. Dugaiczyk, A., et al., J. Mol. Biol. 96: 171 (1975); Helfman, D.M., et al., Focus vol. 6, No. 1 (Bethesda Research Laboratories 1984). The ligation was carried with T4 DNA ligase at 0.05 units per microliter of ligation mix at 14°C for 48 hours. The ligation mixture was then transformed into competent E. coli JM109 cells (on deposit with the American Type Culture Collection, 12301 Parklawn Dr., Rockville, Maryland 20852 USA under Accession No. 53323), using the calcium chloride procedure. Maniatis, T., et al., op. cit. Transformants with inserts were identified as ampicillin resistant white colonies in the presence of Xgal. Vieira, J. and Messing, J., Gene Vieira, J. and Messing, J., Gene 19: 259 (1982).

Screening of Transformants

Positive transformants (ampicillin resistant white colonies) were screened for large TBIA DNA inserts (equal to or greater than 10kb) which contained unique restriction sites near or at the middle of the insert. Transformants were lysed by the alkaline lysis plasmid DNA mini-prep method (Maniatis, T., et al., op. cit.) and subjected to restriction enzyme digests. A preferred plasmid from this screening was pCG300 (on deposit with the American Type Culture Collection, 12301 Parklawn Drive, Rockville, Maryland 20852 U.S.A.

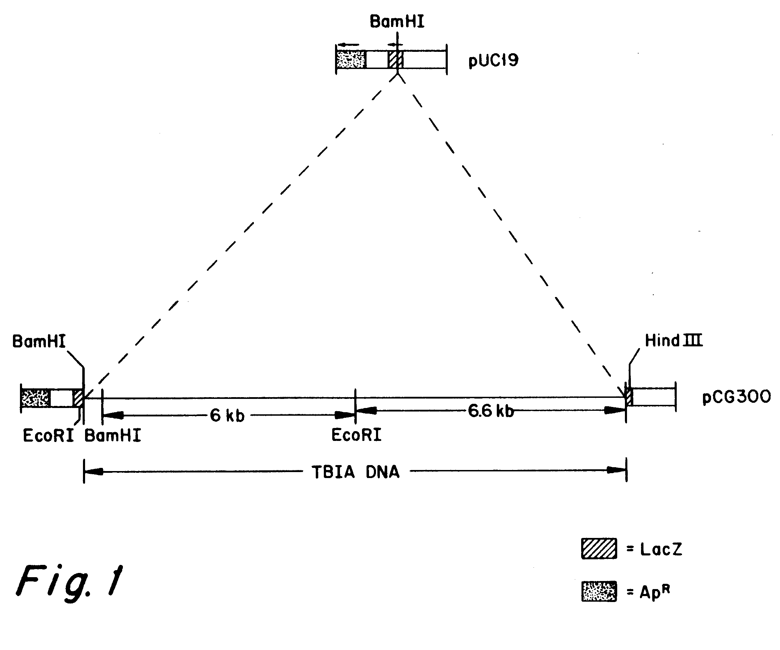

under Accession No. 53329), which contains a 13 kb TBIA DNA Insert with one EcoRI site near the middle of the insert (Fig 1.)

Cloning of Kanamycin Resistance Gene

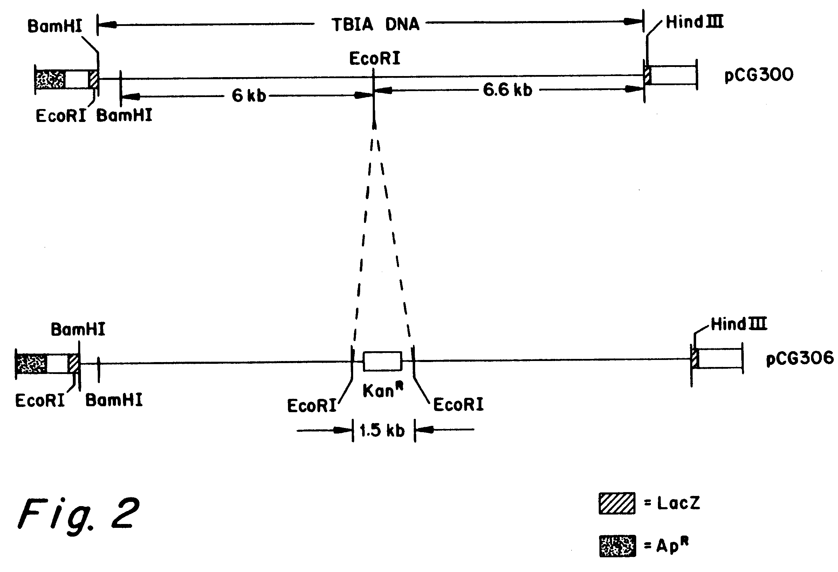

In order to determine that the EcoRI site in the TBIA insert of pCG300 is not located in a critical TBIA gene, a kanamycin resistance cassette (Pharmacia 1984 Catalogue Molecular Biologicals, Chemicals and Equipment for Molecular Biology p. 74) was cloned into the site and this DNA transformed into TBIA, selecting for kanamyhcin resistant integrates. The recombinant plasmid, pCG300, was partially digested with the restriction enzyme EcoRI, and ligated (using T4 DNA ligase), with the EcoRI DNA fragment containing the kanamycin resistance gene from Tn903. Dugaiczyk, A., et al., J. Mol. Biol. 96: 171 (1975); Pharmacia 1984 Catalogue Molecular Biologicals, Chemicals and Equipment for Molecular Biology p. 74. The ligation mixture was incubated at 14°C for 72 hours. The ligation mixture was then transformed into competent E. coli strain SK2267 (on deposit with the American Type Culture Collection, 12301 Parklawn Drive, Rockville, Maryland 20852 USA under Accession No. 53324); transformants were selected on plates containing kanamycin sulfate (50 ug/ml). Kanamycin resistant transformants were screened for the favored construct -- the kanamycin gene cloned into the EcoRI site found in the TBIA DNA insert of pCG300. The new plasmid, pCG306 (Fig. 2), is then used to transform TBIA cells, selecting for kanamycin resistance. The finding of stable transformants shows that the EcoRI site can be used for integration of agricultural chemical genes without compromising the growth of TBIA.

Cloning of promoters from TBIA

In order to insure that foreign genes will be expressed in TBIA, it is preferable to clone regulatory sequences from TBIA for fusion to foreign gene sequences.

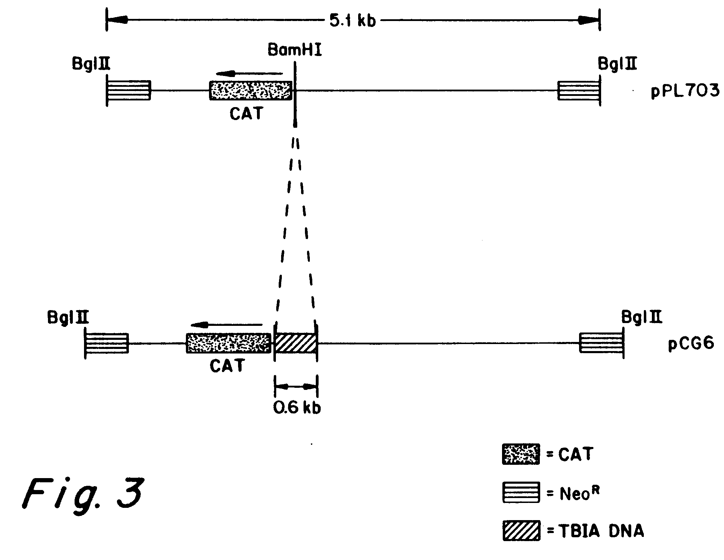

Sau3AI fragments from a partial restriction digest of TBIA chromosomal DNA (see above) were cloned (Maniatis, T., et al., op. cit.) into the BamHI site of a Bacillus promoter cloning plasmid, pPL703 (on deposit with the American Type Culture Collection, 12301 Parklawn Dr., Rockville, Maryland 20852 U.S.A. under Accession No. 53327) which was derived from pPL603. Williams D. et al., J. Bacteriol. 146: 1162-1165 (1981). This plasmid (Fig. 3) contains a structural gene for chloramphenicol acetyltransferase (CAT) which is not expressed due to lack of a promoter. Recombinant plasmids were transformed into competent Bacillus subtilis 62037 (rec E) (on deposit with the American Type Culture Collection, 12301 Parklawn Dr., Rockville, Maryland 20852 U.S.A. under Accession No. 53325). Restriction fragments that promote expression of chloramphenicol acetyltransferase gene were identified by selecting chloramphenicol resistant colonies were obtained, and their chloramphenicol acetyltransferase activities were measured. Shaw W. V., in Methods in Enzymol. 43: 737-755 (Academic Press 1975). The transformants contained recombinant plasmids with TBIA DNA insertions ranging from 0.4 to 2 kilobases and chloramphenicol acetyltranferase activity ranging from 49 to 1026 nmole/min/mg protein. The plasmid pCG6 (Fig. 3) (on deposit with the American Type Culture Collection, 12301 Parklawn Dr., Rockville, Maryland 20852 U.S.A. under Accession No., 53328) with the highest promoter activity for chloramphenicol acetyltransferase is the preferred source of regulatory signals for the expression in TBIA of desired agricultural chemical proteins, e.g. Bacillus thuringiensis insecticidal endotoxin.

Expression of B. thuringiensis Delta-Endotoxin from a Cxc Promoter

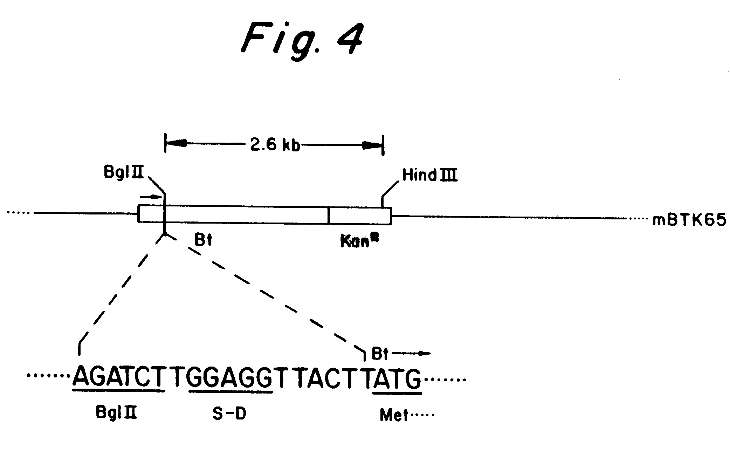

The M13 vector mBTK65 (on deposit with the American Type Culture Collection, 12301 Parklawn Dr., Rockville, Maryland 20852 U.S.A. under Accession No. 53326) contains a truncated B. thuringiensis delta-endotoxin gene fused to a kanamycin resistance gene in such a manner that both insecticidal activity and kanamycin are expressed. The fusion sequence is bounded by a BglII site 5-prime to the Shine-Delgarno (ribosome binding site) sequence and a HindII site 3-prime to the kanamycin resistance gene (Fig. 4). This fusion hybrid sequence can be ligated 3-prime to a Cxc promoter (as in pCG6) to bring expression of both activities under Cxc control. The procedure is as follows: Phage mBTK65 replicative form (RF) DNA is prepared from ATCC No. 53326 by standard methods. Barnes, W.M., et al., in Methods in Enzymology, 101: 98-122 (Academic Press 1983). The purified DNA is cut sequentially with the restriction enzymes BglII and HindIII to liberate a 2.64 kb fragment, which is isolated and purified by preparative agarose electrophoresis as described in Maniatis, T., et al., op. cit. The purified fragment is then blunted by treating with the klenow fragment of DNA polymerase as described in Maniatis, T., et al., op. cit.

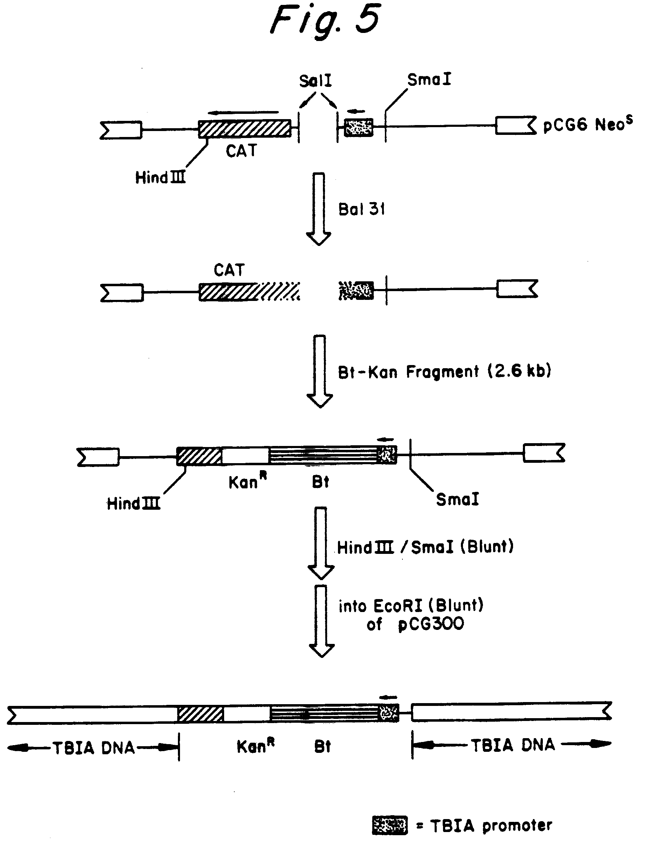

In order to optimize the translation of the delta endotoxin-kanamycin resistance fusion gene, the promoter segment of pCGC6 is treated with the exonuclease Bal-31 to generate multiple 3-prime termini for fusion to the Shine-Dalgarno sequence of the gene fusion. Roberts, T.M., et al., Proc. Natl. Acad. Sci. USA, 76: 760-764 (1979); Deans, R., et al., in Recombinant DNA Techniques, 2: 2-6 (The University of Michigan 1981). In order to select kanamycin resistance generated by the fusion hybrid, it is necessary to inactivate the neomycin (Kanamycin) resistance element already present in pCG6. This is done by cutting pCG6 with BglII endonuclease, which cuts within the neomycin resistance gene; the BglII ends are then filled in with the klenow fragment of DNA polymerase and the blunted vector is recircularized with T4 ligase. Maniatis, T., et al., op. cit. The ligated DNA is transformed into B. subtilis strain 62037 as before, selecting chloramphenicol resistance and screening for kanamycin sensitivity, indicating that a frameshift has resulted in inactivation of the neomycin resistance element. This plasmid is designated pCG6 Neo$^s$. Following digestion of pCG6 Neo$^s$ with SalI, the DNA is subjected to Bal-31 as described (Deans, R., op cit.) (Fig. 5) to generate a family of deletions, whose pattern is monitored by gel electrophoresis. The heterogeneous plasmid fragment population is then ligated with the blunted BGIII-HindIII fragment, above. The ligated DNA is transformed into B. subtilis 62037 (rec E), selecting for kanamycin resistance. Since the expression of kanamycin rsistance in the gene fusion is dependent on the transcription and translation of the 5-prime delta-endotoxin gene, only favorable configurations of promoter and the delta-endotoxin gene fusion will give rise to kanamycin resistance. The kanamycin resistance transformants are screened by standard recombinant DNA techniques (Maniatis, T., et al., op. cit.) to verify the restriction map. The expression of delta-endotoxin activity is monitored by subjecting freshly-hatched larvae of the tobacco hornworm (Carolina Biologicals) to samples of the colony incorporated into their standard diet. Kanamycin resistant transformants found to produce effective levels of larvae toxicity are grown up and their plasmid DNA extracted. The plasmid DNA is cut by SmaI and HindII restriction endonucleases which cut out the promoter-gene fusion region (cf. Fig 5) and the ends blunted as before. This fragment is then cloned into the integration vector pCG300 at the blunted EcoRI site by standard methods (Maniatis, T., et al., op. cit.). The ligated DNA is transformed as previously into E. coli SK267, selecting for kanamycin resistance. Transformants from the latter procedure are screened as before for insecticidal activity and expected restriction map. Transformants giving the correct restriction map and insecticidal activities are then grown up for plasmid DNA preparation and subsequent transformation of TBIA.

Preparation of TBIA protoplasts

TBIA protoplasts are prepared by the following method:
1) inoculate TBIA from frozen glycerol stock into S8 medium;
2) aerate the culture at 30°C until mid-exponential;
3) add 2% glucose, 0.1% glycine to the culture, and continue aeration overnight (approximately 178 hours);
4) spin down 20 ml of culture in sterile tubes at 8000 rpm for 5 minutes at 4°C, was once in 10 ml of SMMC buffer (sorbitol o.5M - 20 mM maleate - 20mM MgCl$_2$ - 20 mM CaCl$_2$ (pH 7.0) (Yoshihama M., et al., J. Bacteriol. 162: 591-597 (1985)) and suspend in 2 ml of SMMC buffer containing 2.5 mg/ml lysozyme;
5) incubate at 30°C for 2-3 hours, and check for the formation of protoplasts under a microscope; and
6) harvest cells by centrifugation at 6000 rmp for 10 minutes at 4°C, was once with 5 ml of SMMC buffer, and suspend in 1 ml of SMMC buffer.

Transformation of TBIA protoplasts

A 20 ml exponential culture of TBIA growing in S8 medium is pretreated with 2% glucose and 0.1% glycine overnight (approximately 17 hours), and protoplasts are prepared as described above. Samples (0.3 ml) of protoplasted cells are placed in tubes, and integrative plasmid DNA carrying the TBIA promoter and B. thuringiensis delta endotoxin fusion gene (1 ug DNA in 10 ul of 0.5 M sorbitol (pH 7.0)) is added. Then 0.7 ml of 25% PEG in SMMC buffer is added, followed by gentle mixing. After incubation at 25°C for 5 min., the mixture is diluted 10 fold with SMMC, centrifuged as above, suspended in 1 ml of SSC broth (S8 broth with 0.5 M sorbitol (pH 7.0)), and incubated with shaking at 30°C for 2 hrs.

The transformed protoplasts (0.1 ml) are plated onto cellulose acetate membrane filters on top of nonselective regeneration plates (SSC), and incubated for 2-3 days at 30°C to allow expression of kanamycin resistance. The filters are then transferred to selective regeneration plates (SSC + kanamycin (50 ug/ml)), and incubated further at 30°C to select for kanamycin resistant transformants.

Test for Larvacidal Activity of Hybrid TBIA in Corn

Kanamycin resistant transformants (above) are then grown in S8 medium plus 50 ug/ml kanamycin and portions of the culture tested for larvacidal activity in vivo as described above. The larvacidal isolates are then inoculated into corn seedlings (FR 632, Illinois Foundation Seeds). Inoculations are performed by piercing 2- 3 week old seedlings with a pointed sterile scalpel which has been dipped in a colony to be tested. Periodically during the life cycle of the plant, xylem sap samples are removed and tested for kanamycin resistant hybrids which are larvacidal in vivo. As stated elsewhere in this document, isolates that routinely show colonization of the corn without disease symptoms, and which express the agricultural chemical (B. thuringiensis endotoxin) are selected for tests of in vivo efficacy. The latter test is performed by challenging inoculated corn plants with freshly hatched larvae of the corn earworm or the European corn borer.

While the processes and products of the present invention have been described primarily in reference to hybrids of bacteria capable of fixing nitrogen or producing avermectins, it is to be understood that, with appropriate changes in the screening procedure, hybrids capable of producing other agricultural chemicals, such as insecticides, herbicides, antifungal agents, antibacterial agents, antiviral agents, plant growth regulators, and solubilized phosphates can be prepared in accordance with the present invention. Moreover, while the foregoing examples illustrate the inclusion of genes for production of one agricultural chemical in the hybrid, it is envisioned that genes for production of two or more compatible products can be included in hybrids found in accordance with the present invention. Thus, fusion of hybrids formed in accordance with the present invention with other agricultural-chemical-producing organisms, initial fusion with microorganisms known to produce more than one agricultural chemical, or initial fusion conducted with more than one agricultural-chemical-producing microorganism would yield such multi-chemical-producing organisms. Similarly, two or more genes for the production of agricultural chemicals can be cloned into an infecting microorganism, using recombinant DNA techniques. For example, the genes for the delta-endotoxins of Bacillus, thuringiensis var. kurstaki and Bacillus thurigiensis var. tenebrionis could be cloned into an infecting microorganism. In addition, the scope of applicable plant hosts can be expanded by combining the genetic material of two or more infecting microorganisms.

It will be apparent to those skilled in the art that various modifications and variations can be made in the processes and products of the present invention. Thus, it is intended that the present invention cover the modifications and variations of this invention provided that they come within the scope of the appended claims and their equivalents.

## Claims

1. A method of producing hybrid-agricultural-chemical-producing microorganisms capable of entering into endosymbiotic relationships with a plant host comprising:
   (A) in any convenient order:
      (1) identifying said plant host; and
      (2) identifying an infecting microorganism which infects said plant host;
   (B) preparing a vector capable of being transferred into and replicating in said infecting microorganism;
   (C) preparing an expression module capable of directing the production of an agricultural chemical by said infecting microorganism;
   (D) placing said expression module in said vector to create an expression vector capable of being transferred into and replicating in said infecting microorganism, said expression vector being capable of directing the production of said agricultural chemical by said infecting microorganism;
   (E) transforming said infecting microorganism with said expression vector to produce hybrid microorganisms;
   (F) selecting for said hybrid microorganisms;
   (G) selecting or screening serially from said selected hybrid microorganisms and in any convenient order:
      (1) a subgroup comprising those hybrid microorganisms which manifest the ability to interact with plant tissue in the manner in which said infecting microorganism interacts with plant tissue during the initial phase of infection in said plant host;
      (2) a subgroup comprising those hybrid microorganisms which, upon application to said host, do not create manifestations of disease; and

(3) a subgroup comprising those hybrid microorganisms having the ability to produce said agricultural chemical if not previously selected for; and

(H) selecting hybrid agricultural-chemical-producing microorganisms capable of entering into endosymbiotic relationships with a plant host by selecting from the products of the last performed step of steps (G)(1) to (G)(3) those hybrid microorganisms capable of improving the performance of said plant host under conditions wherein said performance would be improved by direct application of said agricultural chemical or said agricultural-chemical-producing microorganism to said plant host.

2. The method according to Claim 1, wherein said step (C) comprises:

preparing a portable DNA sequence containing the structural gene or genes for said agricultural chemical;

cloning said portable DNA sequence into a vector containing transcription and translation control elements for said portable DNA sequence, said control elements being operable in said infecting microorganism, to create the expression module; and

recovering said expression module.

3. The method according to Claim 1 or 2, wherein

in step (B), an integration vector containing an integration sequence is prepared which is capable of integrating into the genome of said infecting microorganism;

in step (D), said expression module is placed within said integration sequence of said integration vector, thereby producing a modified integration vector capable of integrating into the genome of said infecting microorganism; and

in step (E), said infecting microorganism is transformed with said modified integration vector.

4. The method according to any of claims 1 to 3, wherein said expression module further comprises a DNA sequence that codes for a selectable trait in said infecting microorganism.

5. The method according to claim 1 or 3, wherein said step (c) comprises:

preparing a vector containing a promoter that is operable in said infecting microorganism;

placing into said vector a portable DNA sequence containing the structural gene or genes for the production of said agricultural chemical and containing transcription and translation control elements other than a promoter, said control elements being operable in said infecting microorganism, to produce an expression vector containing an expression module, said expression module comprising said portable DNA sequence and said control elements and said expression module being operable in said infecting microorganism; and

recovering said expression module from said expression vector.

6. The method according to Claim 5 comprising the further step of placing into said vector a DNA sequence that codes for a selectable trait in said infecting microorganism, wherein said expression module further comprises said DNA sequence that codes for said selectable trait.

7. The method according to Claim 6, wherein said DNA sequence that codes for a selectable trait in said infecting microorganism is within said expression module.

8. The method according to any of Claims 1 to 7, wherein said infecting microorganism is a bacterium.

9. The method according to Claim 8 , wherein said bacterium is a species of the genus Corynebacterium and/or Clavibacter.

10. The method according to any of Claims 1 to 9, wherein said plant host is of the Gramineae family.

11. The method according to Claim 10, wherein said plant host is bermudagrass, sugar cane or sorghum.

12. The method according to any of Claims 1 to 9, wherein said plant host is corn.

13. The method according to Claim 8, wherein said bacterium is Clavibacter xyli subsp. cynodontis and/or subsp. xyli, and said plant host is corn.

14. The method according to any of Claims 1 to 13, wherein said agricultural chemical is the delta endotoxin of <u>Bacillus thuringiensis</u>.

15. The method according to Claim 3, wherein said integration vector is pCG300.

16. The method according to Claim 5, wherein the said vector containing a promoter is pCG6.

*Fig. 1*

Fig. 2

BamHI ──────── TBIA DNA ──────── HindIII

EcoRI

EcoRI BamHI

6 kb

6.6 kb

pCG300

BamHI

EcoRI BamHI

EcoRI    Kan^R    EcoRI

1.5 kb

HindIII

pCG306

= LacZ

= Ap^R

Fig. 3

EP 0 583 675 A1

EP 0 583 675 A1

# Fig. 4

# Fig. 5

## DOCUMENTS CONSIDERED TO BE RELEVANT

| Category | Citation of document with indication, where appropriate, of relevant passages | Relevant to claim | CLASSIFICATION OF THE APPLICATION (Int.Cl.4) |
|---|---|---|---|
| A | ENGINEERED ORGANISMS IN THE ENVIRONMENT 1985 pages 40 - 46 WATRUD, L.S., ET AL. 'Cloning of the Bacillus thuringiensis subsp. kurstaki delta-endotoxin gene into Pseudomonas flourescens: Molecular biology and ecology of an engineered microbial pesticide' * the whole document * | 1-16 | C12N15/74 C12N15/31 A01N63/00 |
| A | EP-A-0 125 468 (CROP GENETICS INTERNATIONAL) 21 November 1984 * the whole document * | 1-16 | |
| A | EP-A-0 130 047 (AGRIGENETICS) 2 January 1985 * page 11, paragraph 2; example 9 * | 1-16 | |
| | | | **TECHNICAL FIELDS SEARCHED** (Int.Cl.4) |
| | | | C12N A01N C07K |

The present search report has been drawn up for all claims

| Place of search | Date of completion of the search | Examiner |
|---|---|---|
| THE HAGUE | 3 November 1993 | MADDOX, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)